# FASCICULE DE BREVET EUROPEEN

(11) **EP 0 921 119 B1**
(45) Date de publication et mention de la délivrance du brevet: **02.07.2003**
(21) Numéro de dépôt: 98402926.4
(22) Date de dépôt: 25.11.1998
(51) Int. Cl.: C07D 209/44, C07D 209/52, C07D 209/72, C07D 409/04, C07D 409/14, A61K 31/40, C07D 487/04, C07D 401/14, C07D 405/14

(54) **Nouveaux derives de pyrrole leur procédé de preparation et les compositions pharmaceutiques qui les contiennent**
Pyrrolderivate, Verfahren zu ihrer Herstellung und diese enthaltende pharmazeutische Zusammensetzungen
Pyrrole derivatives, process for their preparation and pharmaceutical compositions containing them

(30) Priorité: 26.11.1997 FR 9714840
(43) Date de publication de la demande: 09.06.1999
(73) Titulaire: LES LABORATOIRES SERVIER, 92200 Neuilly sur Seine (FR)
(72) Inventeur: De Nanteuil, Guillaume, 92150 Suresnes (FR); Portevin, Bernard, 78990 Elancourt (FR); Bonnet, Jacqueline, 75013 Paris (FR); Tordjman, Charles, 92100 Boulogne (FR)

(56) Documents cités:
- WO-A-98/02430
- T. KOBAYASHI ET AL: "Syntheses and spectra properties of norbornadiene-fused heterocycles: 1,3-Diphenyl-4,7-dihydro-4,7-methanobenzen e(c)thiophene, -4,7-methano-2H-isoindole, and 4,7-methanoisobenzofuran" BULLETIN OF THE CHEMICAL SOCIETY OF JAPAN, vol. 66, no. 9, 1993, pages 2707-2713, XP002074575
- K. MONNIER ET AL: "Modulation de la régiochemie de la réaction du fluoroborate de 2-benzoyl-1,2-dihydroisoquinaldonitrile sur l'indène" BULLETIN DES SOCI T S CHIMIQUES BELGES, vol. 101, no. 2, 1992, pages 109-112, XP002074572
- W. E.MCEWEN ET AL: "Substituent effects in the cycloaddition reactions of Reissert hydrofluoroborate salts with alkene" JOURNAL OF ORGANIC CHEMISTRY, vol. 46, no. 8, 1981, pages 1656-1662, XP002074576 EASTON US
- R. HUISGEN ET AL: "Benzonitril-(4-nitro-benzylid) und seine Reaktionen mit CC-Doppel- und CC-Dreifachbindungen" CHEMISCHE BERICHTE, vol. 105, no. 4, 1972, pages 1258-1278, XP002074577
- K. MATSUMOTO ET AL: "Synthesis of 2,3,4-triphenyl-3-azabicyclo(3.2.0)hepta-1 ,4-diene and its novel reaction with dimethyl acetylenedicarboxylate" JOURNAL OF THE CHEMICAL SOCIETY, PERKIN TRANSACTIONS 1, no. 18, 21 septembre 1997, pages 2691-2693, XP002074573 LETCHWORTH GB
- J.W.LOWN ET AL: "Reactions of functionalized 1,3-dipoles" CANADIAN JOURNAL OF CHEMISTRY, vol. 52, no. 5, 1974, pages 798-809, XP002074574
- M. CHRISTL ET AL: "Some valenes of benzannelated five-member heteroarenes - synthesis and NMR spectra" ANGEWANDTE CHEMIE (INTERNATIONAL EDITION), vol. 29, no. 6, 1990, pages 675-677, XP002074578

## Description

Quelques dérivés du pyrrole de structure dihydro ou tétrahydro isoindolique sont décrits dans la littérature. Parmi ceux-ci, on peut relever les composés présentant en positions 1 et 3 soit un groupement phényle (*Bull. Chem. Soc. Jp*., 1993, 66 (9), 2707-2713), soit un groupement phényle substitué (*Chem. Ber*., 1972, 105, 1258-1278), soit un groupement phényle et une isoquinoléine (*J. Org. Chem.,* 1981, 46, 1656 ; *Bull. Soc. Chem. Belges,* 1992, 101 (2), 109-112). Ces composés particuliers sont exposés dans ces documents au travers de leur voie de synthèse ou de leurs caractéristiques spectrales. Aucune activité thérapeutique n'est connue et révélée pour ces dérivés. Des dérivés de structure proche, utiles en tant qu'antagoniste de l'interleukine-1 et du TNF sont décrits dans la demande de brevet WO 98/02430.

L'originalité des composés de l'invention, outre le fait qu'ils soient nouveaux, réside dans leur activité inhibitrice sélective de la cyclo-oxygénase-2 (COX 2) et de la nitrique oxyde synthase inductible (iNOS).

Les prostaglandines (PG) jouent un rôle important dans l'évolution des réactions inflammatoires. Depuis la découverte de Vane en 1971 *(Nature,* 1971, 321, 232-235) qui a relié l'activité des anti-inflammatoires non stéroïdiens (AINS) à l'inhibition de la voie de la cyclo-oxygénase de la cascade arachidonique, l'inhibition de la production des PG constitue la cible principale dans la découverte de composés à activité anti-inflammatoire.

Cependant, les composés actifs sur la douleur et l'inflammation induites par les PG sont aussi inhibiteurs de processus physiologiques régulés par les PG indépendamment de la réaction inflammatoire : ainsi, présentent-ils des effets secondaires indésirables tels que ulcères gastriques et/ou atteintes rénales.

La découverte en 1991 (*J. Biol. Chem.,* 1991, 266, 12866-12872 ; *Proc. Natl. Acad. Sci. USA*, 1991, 88, 2692-2696) d'une isoenzyme de cyclo-oxygénase (COX) a permis d'établir la différence entre la COX constitutive (COX 1), largement distribuée dans l'organisme, en particulier dans l'estomac et le rein, et la COX inductible (COX 2), dont la synthèse est induite par des stimuli inflammatoires et mitogènes. L'hypothèse a donc été avancée qu'un inhibiteur sélectif de la COX 2 pourrait être un dérivé anti-inflammatoire puissant, tout en étant dénué d'effets secondaires gastro-intestinaux et/ou rénaux.

L'interleukine 1β (IL1β) est produite par les macrophages et constitue le point central de nombreux processus inflammatoires. En particulier, l'IL1β stimule les cellules qui synthétisent et expriment la COX 2 pour fournir des PG. L'IL1β est également responsable de l'expression et de la synthèse de la NO synthase inductible et des protéases qui participent à la dégradation de la matrice extracellulaire du cartilage.

Les processus inflammatoires médiés par les COX sont communs à de nombreuses pathologies. Ils jouent un rôle important en rhumatologie et en particulier dans l'arthrite rhumatoïde et l'arthrose. L'inhibition de la COX 2 a été proposée pour limiter les réactions inflammatoires propres à l'évolution de ces pathologies. L'inhibition de l'IL1β constitue également une cible permettant de réguler d'une part l'inflammation et d'autre part la dégradation articulaire caractéristique de ces pathologies.

Les composés de la présente invention, en plus de leur nouveauté, se sont révélés être des inhibiteurs spécifiques de la COX 2, de l'IL1β et de la iNOS, ce qui les rend potentiellement utiles pour le traitement des processus inflammatoires intervenant notamment dans les maladies rhumatismales telles que l'arthrose, l'arthrite rhumatoïde, mais aussi dans l'athérosclérose, le cancer, etc...

Plus particulièrement, la présente invention concerne les composés de formule (I) : 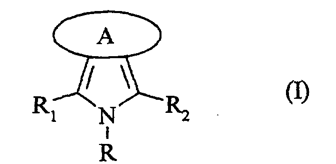 dans laquelle :
- **R**: représente :
. un atome d'hydrogène,
. un groupement alkyle (C₁-C₆) linéaire ou ramifié, éventuellement substitué par un ou plusieurs groupements, identiques ou différents, choisis parmi hydroxy, alkoxy (C₁-C₆) linéaire ou ramifié, carboxy et alkoxycarbonyle (C₁-C₆) linéaire ou ramifié,
. un groupement amino éventuellement substitué par un ou deux groupements, identiques ou différents, choisis parmi alkyle (C₁-C₆) linéaire ou ramifié, alkoxycarbonyle (C₁-C₆) linéaire ou ramifié, arylalkoxy (C₁-C₆) carbonyle linéaire ou ramifié, alkylsulfonyle (C₁-C₆) linéaire ou ramifié, et arylsulfonyle,
. ou un groupement acyle (C₁-C₆) linéaire ou ramifié,
- **R**_{**1**}**, R**_{**2**}: identiques ou différents, représentent chacun indépendamment l'un de l'autre un groupement aryle, ou hétéroaryle, chacun de ces groupements pouvant être éventuellement substitué par un ou plusieurs groupement, identiques ou différents, choisis parmi :
- atome d'halogène,
- groupement alkyle (C₁-C₆) linéaire ou ramifié, éventuellement substitué par un ou plusieurs atomes d'halogène, groupements hydroxy, alkoxy (C₁-C₆) linéaire ou ramifié, amino, ou alkoxycarbonyl (C₁-C₆) linéaire ou ramifié,
- groupement alkoxy (C₁-C₆) linéaire ou ramifié éventuellement substitué par un groupement amino, lui-même éventuellement substitué par un ou deux groupements, identiques ou différents, alkyle (C₁-C₆) linéaire ou ramifié,
- groupement trihalogénoalkoxy (C₁-C₆) linéaire ou ramifié,
- groupement acyle (C₁-C₆) linéaire ou ramifié,
- groupement hydroxy, nitro, cyano, mercapto, carboxy,
- groupement amino éventuellement substitué par un ou deux groupements, identiques ou différents, choisis parmi alkyle (C₁-C₆) linéaire ou ramifié, alkylcarbonyle (C₁-C₆) linéaire ou ramifié, et alkylsulfonyle (C₁-C₆) linéaire ou ramifié,
- groupement alkoxycarbonyle (C₁-C₆) linéaire ou ramifié,
- groupement alkylthio (C₁-C₆) linéaire ou ramifié,
- groupement sulfonyle substitué par un groupement alkyle (C₁-C₆) linéaire ou ramifié ou un groupement amino lui-même éventuellement substitué par un ou deux groupements alkyle (C₁-C₆) linéaire ou ramifié, identiques ou différents, insaturé, et contenant 1, 2 ou 3 hétéroatomes, identiques ou différents, choisis parmi azote, oxygène et soufre, éventuellement substitué par un ou plusieurs groupements, identiques ou différents, choisis parmi halogène, alkyle (C₁-C₆) linéaire ou ramifié, et alkoxy (C₁-C₆) linéaire ou ramifié,
- **A**: représente avec les atomes communs du pyrrole :
- un cycloalkyle (C₃-C₁₂), monocyclique ou bicyclique, saturé ou insaturé (à la condition que la(les) insaturation(s) présente(s) dans le cycloalkyle, ne lui confère(nt) pas un caractère aromatique),
- un hétérocycle saturé de 5 à 7 chaînons contenant un ou deux atomes d'azote,
- ou un oxa-7-bicyclo[2.2.1]heptane,
chacun de ces cycles pouvant être éventuellement substitué par un ou plusieurs groupements, identiques ou différents, choisis parmi :
- atome d'halogène,
- groupement trihalogénométhyle,
- groupement alkyle (C₁-C₆) linéaire ou ramifié, éventuellement substitué par un ou plusieurs atomes d'halogène, ou groupements hydroxy,
- groupement alkoxy (C₁-C₆) linéaire ou ramifié,
- groupement aminoalkyle (C₁-C₆) linéaire ou ramifié,
- groupement cyano,
- groupement aryle, arylalkyle (C₁-C₆) linéaire ou ramifié, la partie aryle de cesdits groupements pouvant être éventuellement substituée par un ou plusieurs groupements, identiques ou différents, choisis parmi halogène, hydroxy, alkyle (C₁-C₆) linéaire ou ramifié, et alkoxy (C₁-C₆) linéaire ou ramifié,
- et groupement sulfonyle substitué par un groupement alkyle (C₁-C₆) linéaire ou ramifié ou un groupement amino lui-même éventuellement substitué par un ou deux groupements alkyles (C₁-C₆) linéaires ou ramifiés, identiques ou différents,
étant entendu que :
. par groupement aryle, on entend un groupement phényle ou napthtyle, et par groupement hétéroaryle, on entend un groupement aryle contenant un, deux ou trois hétéroatomes, d'azote, ainsi que leurs sels d'addition à un acide ou à une base pharmaceutiquement acceptable,
étant entendu que :
- lorsque A, avec les atomes communs du cycle pyrrole, consiste en un cyclohexane ou un norbornène et que R consiste en un atome d'hydrogène, alors R₁ et R₂ ne peuvent pas représenter simultanément un groupement phényle,
- lorsque R₁ représente un groupement phényle, R représente un atome d'hydrogène,
   □ et que R₂ représente un groupement paranitrophényle, alors A, avec les atomes communs du cycle pyrrole, ne peut pas représenter un norbornane,
   □ ou que R₂ représente un groupement 1-isoquinolyle, alors A, avec les atomes communs du cycle pyrrole, ne peut pas représenter un cyclohexane ou un groupement 1,2-indanyle.
étant entendu également que :
- si R₂ représente un groupement 4-pyridyle ou 4-quinolyle, chacun de ces groupements étant éventuellement substitué;
- et R₁ représente un groupement phényle, naphtyle, pyridyle ou quinolyle, chacun de ces groupements étant éventuellement substitué,
- alors A, avec les atomes communs du cycle pyrrole, ne peut pas représenter un groupement cycloalkyle (C₅-C₈) monocyclique, saturé, ou un hétérocycle saturé de 5 à 7 chaînons contenant 1 ou 2 atomes d'azote, chacun de ces groupements étant éventuellement substitué.

D'une façon avantageuse, les composés préférés de l'invention sont ceux de formule (I) dans laquelle :
- R₁ et R₂, identiques ou différents, représentent chacun indépendamment l'un de l'autre, un groupement aryle éventuellement substitué par un ou plusieurs groupements quelconques tels que définis précédemment, et avantageusement par un ou plusieurs atomes d'halogène,
- et A représente, avec les atomes communs du cycle pyrrole, un cycloalkyle (C₃-C₁₂) et avantageusement (C₅-C₈), monocyclique ou bicyclique, saturé ou insaturé mais sans caractère aromatique, éventuellement substitué par un ou plusieurs des substituants quelconques tels que définis précédemment,
ainsi que leurs sels d'addition à un acide ou à une base pharmaceutiquement acceptable.

D'une façon particulièrement avantageuse, les composés préférés de l'invention sont ceux de formule (I) dans laquelle A représente, avec les atomes communs du cycle pyrrole, un cycloalkyle (C₅-C₁₂), et avantageusement (C₅-C₈), bicyclique, saturé ou insaturé, mais sans caractère aromatique, éventuellement substitué par un ou plusieurs groupements quelconques tels que définis précédemment,
ainsi que leurs sels d'addition à un acide ou à une base pharmaceutiquement acceptable.

Selon une autre variante avantageuse de l'invention, les composés préférés sont ceux de formule (I) dans laquelle :
- R₁ et R₂, identiques, représente un groupement hétéroaryle éventuellement substitué par un ou plusieurs groupements quelconques tels que définis précédemment,
- et A représente, avec les atomes communs du cycle pyrrole, un cycloalkyle (C₅-C₁₂) et avantageusement (C₅-C₈), bicyclique, saturé ou insaturé, mais sans caractère aromatique, éventuellement substitué par un ou plusieurs groupements quelconques, tels que définis précédemment,
ainsi que leurs sels d'addition à un acide ou à une base pharmaceutiquement acceptable.

Enfin, d'une façon très avantageuse, les composés préférés de l'invention sont les composés de formule (I) qui sont :
■ le 1,3-di-(4-Fluorophényl)-4,5,6,7-tétrahydro-2*H*-isoindole,
■ le 1,3-Diphényl-5,6-diméthyl-4,5,6,7-tétrahydro-2*H*-isoindole,
■ le 1,3-Diphényl-4,7-méthano-4,5,6,7-tétrahydro-2*H*-isoindole,
■ le 1,3-di-(4-Fluorophényl)-2,4,5,6-tétrahydrocyclopenta[*c*]pyrrole,
■ le 1,3-Diphényl-4,5,6,7-tétrahydro-4,7-éthano-2*H*-isoindole,
■ et le 1,3-di-(4-Fluorophényl)-4,7-méthano-4,5,6,7-tétrahydro-2*H*-isoindole,
et leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la diéthylamine, la tertbutylamine, l'arginine, la lysine, etc...

L'invention s'étend également au procédé de préparation des composés de formule (I), caractérisé en ce que l'on utilise comme produit de départ
* *soit des composés de formule (II*/*a) :* 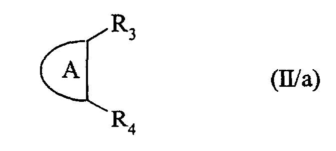
   - R₃: représente un groupement cyano,
   - R₄: représente un groupement alkoxycarbonyle (C₁-C₆) linéaire ou ramifié,
   composés de formule (II/a) que l'on soumet à l'action d'un magnésien de formule (III) :

   R₁ Mg Y (III)

   dans laquelle :
   R₁ est tel que défini dans la formule (I),
   et Y représente un atome d'halogène tel que le brome ou le chlore,
   pour conduire aux composés de formule (I/a), cas particulier des composés de formule (I) : 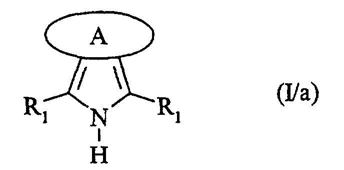 dans laquelle R₁ et A ont la même définition que dans la formule (I),
* *soit des composés de formule (II*/*b) :* 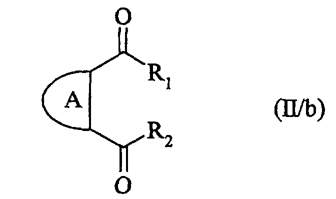 dans laquelle A, R₁ et R₂ sont tels que définis dans la formule (I), que l'on condense :
   ◆ **soit,** en présence d'un acide organique comme l'acide acétique, avec un composé de formule (IV) :

      R-NH₂ (IV)

      dans laquelle R a la même signification que dans la formule (I), à la condition que R soit différent d'un atome d'hydrogène, pour conduire aux composés de formule (I/b), cas particulier des composés de formule (I) : 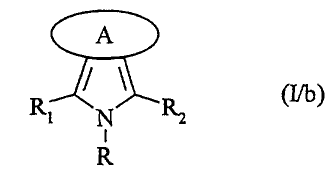 dans laquelle :
      R₁ et R₂, identiques ou différents, ont la même signification que dans la formule (I),
      A est tel que défini dans la formule (I) et R est tel que défini dans la formule (I) à la condition qu'il ne constitue pas un atome d'hydrogène,
      composés de formule (I/b) que l'on soumet éventuellement à l'action d'un agent de déalkylation, de désamination ou de déacylation suivant la nature du groupement R, pour conduire aux composés de formule (I/c), cas particulier des composés de formule (I) : 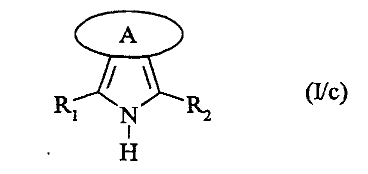 dans laquelle R₁, R₂ et A sont tels que définis précédemment,
   ◆ **soit** avec du formiate d'ammonium HCO₂⁻ NH₄⁺, pour conduire directement aux composés de formule (I/c), cas particulier des composés de formule (I) : 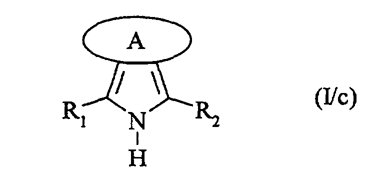 dans laquelle R₁ et R₂ (identiques ou différents), et A ont la même signification que dans la formule (I),
   l'ensemble des composés (I/a), (I/b) et (I/c) pouvant éventuellement subir une réaction de réduction catalytique, dans les cas où le cycle A comporte au moins une insaturation, pour conduire aux composés de formule (I/d), cas particulier des composés de formule (I) : 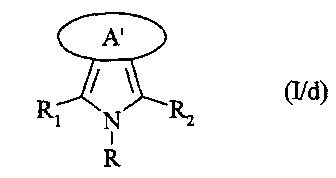 dans laquelle R, R₁ et R₂ ont la même signification que dans la formule (I) et A' représente un cycloalkyle mono ou bicyclique (C₃-C₁₂) saturé (éventuellement substitué),
composés de formule (I/a) à (I/d), que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

Les composés de formule (II), ((II/a) et (II/b)), sont, soit des composés commerciaux, soit obtenus pour les composés de formule (II/a), selon les conditions décrites par *J. Am. Chem. Soc*., 1962, 84, 2196 et pour les composés de formule (II/b) par une réaction de Diels-Alder entre une dicétone insaturée et un diène.

L'invention s'étend aussi aux compositions pharmaceutiques renfermant comme principe actif au moins un composé de formule (I) seul ou en combinaison avec un ou plusieurs excipients inertes, non toxiques et pharmacologiquement acceptables. Parmi les compositions pharmaceutiques selon l'invention, on pourra citer plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublingaux, les gélules, les capsules, les suppositoires, les crèmes, les pommades, les gels dermiques, les préparation injectables ou buvables, les gouttes oculaires ou nasales, etc...

L'invention comprend également, pour leur utilisation comme médicament, les composés suivants, cas particulier des composés de formule (I), pour laquelle :
- lorsque R₁ et R₂ sont identiques et représentent un groupement phényle, et que R suivants, cas particulier des composés de formule (I), pour laquelle :

- lorsque R₁ et R₂ sont identiques et représentent un groupement phényle, et que R représente un atome d'hydrogène, alors A, avec les atomes communs du cycle pyrrole, représente un cyclohexane,
- et lorsque R₁ et R₂ sont identiques et représentent un groupement phényle, et que R représente un atome d'hydrogène, alors A, avec les atomes communs du cycle pyrrole, représente un norbornène,
- et lorsque R₁ représente un groupement phényle, R₂ représente un groupement 1-isoquinolyle, et que R représente un atome d'hydrogène, alors A, avec les atomes communs du cycle pyrrole, représente un cyclohexane,
- et lorsque R₁ représente un groupement phényle, R₂ représente un groupement paranitrophényle, et que R représente un atome d'hydrogène, alors A, avec les atomes communs du cycle pyrrole, représente un norbornane.

L'invention s'étend de même aux compositions pharmaceutiques renfermant comme principe actif au moins un composé correspondant à une de ces quatre structures, cas particuliers des composés de formule (I), telles que définies ci-dessus, seul ou en combinaison avec un ou plusieurs excipients inertes, non toxiques et pharmacologiquement acceptables pour utilisation comme inhibiteur de la cyclo-oxygénase-2, de l'interleukine 1β et de la nitrique oxyde synthase inductible.

La posologie utile est adaptable selon la nature et la sévérité de l'affection, la prise de traitements éventuels associés, la voie d'administration ainsi que selon l'âge et le poids du patient. Cette posologie s'échelonne de 0,1 mg à 1 g, en une ou plusieurs prises par jour.

Les exemples suivants illustrent l'invention mais ne la limitent en aucune façon. Les exemples 36 et 37 ne font pas partie de l'invention et ne sont décrits qu'à titre indicatif.

Les produits de départ utilisés sont des produits connus ou préparés selon des modes opératoires connus. Les différents stades conduisent à des intermédiaires de synthèse utiles pour la préparation des composés de l'invention. spectrométrie de masse...).

### PREPARATION :

Les produits des diverses préparations, utiles pour la synthèse, en tant que produit de départ, des composés de l'invention décrits dans les différents exemples, ont été obtenus par réaction entre un diène et un diénophile selon les conditions opératoires décrites notamment dans *J. Am. Chem. Soc*., 1940, 62, 56-61.

### PREPARATION 1 : 1,2-Diméthyl-4,5-dibenzoyl-cyclohexène

Le produit est obtenu par réaction entre un diène, le 2,3-Diméthyl-1,3-butadiène, et un diénophile le 1,4-Diphényl-2-butène-1,4-dione.

### PREPARATION 2 : 4,5-Dibenzoyl-cyclohexène

Le produit est obtenu par réaction entre un diène, le 1,3-butadiène et le diénophile de la préparation 1.

### PREPARATION 3 : 5,6-Dibenzoyl-bicyclo[2.2.1]-heptène-2

Le produit est obtenu par réaction entre un diène, le cyclopentadiène et le diénophile de la préparation 1.

### PREPARATION 4 : 5,6-Dibenzoyl-bicyclo[2.2.2]-octène-2

Le produit est obtenu par réaction entre un diène, le 1,3-cyclohexadiène, et le diénophile de la préparation 1.

### PREPARATION 5 : 5,6- (4-Fluorobenzoyl)-bicyclo[2.2.1]-heptène-2

Le produit est obtenu par réaction entre le diène de la préparation 3 et un diènophile, le 1,4-di-(4-fluorophényl)-2-butène-1,4-dione.

### PREPARATION 6 : 4,5-di-(4-Méthoxybenzoyl)-cyclohexène

Le produit est obtenu par réaction entre le 1,3-butadiène et le 1,4-di-(4-méthoxyphényl)-2-butène-1,4-dione.

### PREPARATION 7 : 4,5-di-(4-Chlorobenzoyl)-cyclohexène

Le produit est obtenu par réaction entre le diène de la préparation 6 et le 1,4-di-(4-chlorophényl)-2-butène-1,4-dione.

### PREPARATION 8 : 2-(4-Fluorobenzoyl)-3-[(4-méthylsulfonyl)benzoyl]-bicyclo[2.2.1]-heptane

Le produit de la préparation 5 est traité dans les conditions de l'exemple 9, puis mis en présence de thiométhylate de sodium dans du diméthylsulfoxyde selon des conditions opératoires classiques. Le produit obtenu est alors soumis à une oxydation permettant d'isoler le produit attendu.

### PREPARATION 9 : 2,3-di-[(4-Méthylsulfonyl)benzoyl]-bicyclo[2.2.1]heptane

Le produit est obtenu comme coproduit lors de la synthèse du composé de la préparation 8.

### PREPARATION 10 : 4,5-di-[4-(1H-Imidazolyl)benzoyl]cyclohexène

Le produit est obtenu par réaction entre le diène de la préparation 6 et le 1,4-di-(4-Fluorophényl)-2-butène-1,4-dione, suivie d'un traitement par de l'imidazole en présence de potasse, dans le diméthylsulfoxyde.

### PREPARATIQN 11 : 5,6-di-(4-Piridylcarbonyl)-bicyclo[2.2.1]-heptène-2

Le produit est obtenu par réaction entre le cyclopentadiène, et le 1,4-di-(4-Pyridyl)-2-butène-1,4-dione.

### PREPARATION 12 : 5,6-di-(2,4-Difluorobenzoyl)-bicyclo[2.2.1]-heptène-2

Le produit est obtenu par réaction entre le cyclopentadiène et le 1,4-di-(2,4-Difluorophényl)-2-butène-1,4-dione.

### PREPARATION 13 : 5,6-di-(3,4-Difluorobenzoyl)-bicyclo[2.2.1]-heptène-2

Le produit est obtenu par réaction entre le cyclopentadiène et le 1,4-di-(3,4-difluorophényl)-2-butène-1,4-dione.

### PREPARATION 14 : 5,6-di-[(5-Fluoro)-2-pyridylcarbonyl]-bicyclo[2.2.1]-heptène-2

Le produit est obtenu par réaction entre le cylopentadiène et le 1,4-di-[(5-Fluoro)-2-pyridyl]-2-butène-1,4-dione.

### PREPARATION 15 : 5,6-di-[(6-Fluoro)-3-pyridylcarbonyl]-bicyclo[2.2.1]heptène-2

Le produit est obtenu par réaction entre le cylopentadiène et le 1,4-di-[(6-Fluoro)-3-pyridyl]-2-butène-1,4-dione.

### PREPARATION 16 : 5,6-di - (2-Furylcarbonyl)-bicyclo[2.2.1]-heptène-2

Le produit est obtenu par réaction entre le cyclopentadiène et le 1,4-di-(2-Furyl)-2-butène-1,4-dione.

### PREPARATION 17 : 5,6-di-(2-Thiénylcarbonyl)-bicyclo[2.2.1]-heptène-2

Le produit est obtenu par réaction entre le cyclopentadiène et le 1,4-di-(2-thiényl)-2-butène-1,4-dione.

### PREPARATION 18 : 5,6-di-[(4-Fluoro)-2-nitrobenzoyl]-bicyclo[2.2.1]-heptène-2

Le produit est obtenu par réaction entre le cyclopentadiène et le 1,4-di-[(4-Fluoro)-2-nitrophényl]-2-butène-1,4-dione.

### PREPARATION 19 : 5,6-di-[(4-Fluoro) -3-nitrobenzoyl]-bicyclo[2.2.1]-heptène-2

Le produit est obtenu par réaction entre le cyclopentadiène et le 1,4-di-[(4-Fluoro)-3-nitrophényl]-2-butène-1,4-dione.

### PREPARATION 20 : 5,6-di-(4-Fluorobenzoyl)-bicyclo[2.2.2]-octène-2

Le produit est obtenu par réaction entre le 1,3-cyclohexadiène et le diénophile utilisé dans la préparation 5.

### PREPARATION 21 : 5,6-di-[(4-Fluoro)benzoyl]-oxo-7-bicyclo[2.2.1]-heptène-2

Le produit est obtenu par réaction entre un diène, le furane et le diénophile utilisé dans la préparation 5.

### Exemple 1 : 1,3-di(4-Fluorophényle)-4,5,6,7-tétrahydro-2H-isoindole

### Stade A : Cis -2-cyano-cyclohexane carboxylate de tert-Butyle

46,25 g de *cis*-cyclohexane-1,2-dicarboxylique anhydride sont additionnés lentement à une température inférieure à 25 °C à une solution de 66 ml d'ammoniaque 10 N. Après 18 heures, la solution est acidifiée par addition d'acide chlorhydrique 12 N, entraînant la formation d'un précipité qui est filtré, lavé à l'eau et séché. Les 51 g d'acide *cis-2*-carboxamido-cyclohexane carboxylique ainsi obtenus sont mélangés à 180 ml de pyridine et 75 ml de tert-Butanol. On additionne alors goutte à goutte 115 ml de benzènesulfochlorure en maintenant la température aux environs de 40 °C. Après 12 heures de réaction à température ambiante, la solution est hydrolysée par addition de 600 ml d'eau puis extraite par de l'éther éthylique. Après lavage, les phases organiques sont séchées sur sulfate de calcium et concentrées. Le résidu est distillé et on récupère 55,7 g du produit attendu.

### Stade B : 1,3-di-(4-Fluorophényle)-4,5,6,7-tétrahydro-2H-isoindole

A 80 ml d'une solution 2M de bromure de 4-Fluoro-phénylmagnésium dans l'éther éthylique sont additionnés goutte à goutte, à 35 °C, une solution de 14,75 g du composé du stade A dilué dans 30 ml d'éther éthylique. Après 2 heures, la réaction est refroidie et hydrolysée par 20 ml d'une solution saturée en chlorure d'ammonium. Après filtration et lavage à l'éther éthylique, les phases organiques sont rassemblées et séchées sur sulfate de calcium puis concentrées. Le produit est isolé par chromatographie sur gel de silice (éluant : cyclohexane / acétate d'éthyle : 97,5 / 2,5). Après cristallisation, 2 g du produit sont obtenus.

| *Microanalyse élémentaire :* | | | |
|---|---|---|---|
| | *C* | *H* | *N* |
| *% calculé* | *77,66* | *5,54* | *4,53* |
| *% trouvé* | *77,26* | *5,52* | *4,42* |

### Exemple 2 : 1,3-Diphényl-4,5,6,7-tétrahydro-2H-isoindole

Le produit attendu est obtenu selon le procédé décrit dans l'exemple 1 en utilisant au stade B le bromure de phénylmagnésium en tant que réactif.

| *Microanalyse élémentaire :* | | | |
|---|---|---|---|
| | *C* | *H* | *N* |
| *% calculé* | *87,87* | *7,00* | *5,12* |
| *% trouvé* | *87,87* | *7,28* | *5,37* |
| **Point de fusion : 146 °C.** | | | |

### Exemple 3 : 1,3-di(4-Méthylthiophényl)-4,5,6,7-tétrahydro-2H-isoindole

Le produit attendu est obtenu selon le procédé décrit dans l'exemple 1 en utilisant au stade B le bromure de 4-Methylthiophénylmagnésium en tant que réactif.

| *Microanalyse élémentaire :* | | | | |
|---|---|---|---|---|
| | *C* | *H* | *N* | *S* |
| *% calculé* | *72,28* | *6,34* | *3,83* | *17,54* |
| *% trouvé* | *72,11* | *6,40* | *4,07* | *17,40* |
| **Point de fusion : 174 °C.** | | | | |

### Exemple 4 : 1,3-Diphényl-2,4,5,6-tétrahydrocyclopenta[c]pyrrole

### Stade A : Cis-2-cyano-cyclopentane carboxylate de tert-Butyle

On procède comme dans le stade A de l'exemple 1 en utilisant le cis-cyclopentane-1,2-dicarboxylique anhydride en tant que substrat.

### Stade B : 1,3-Diphényl-2,4,5,6-tétrahydrocyclopenta[c]pyrrole

Le produit attendu est obtenu en faisant réagir le composé du stade A, selon les conditions du stade B de l'exemple 1, avec le réactif utilisé dans l'exemple 2.

| *Microanalyse élémentaire :* | | | |
|---|---|---|---|
| | *C* | *H* | *N* |
| *% calculé* | *87,99* | *6,61* | *5,40* |
| *% trouvé* | *88,12* | *6,70* | *5,38* |
| **Point de fusion : 190 °C.** | | | |

### Exemple 5 : 1,3-Diphényl-4,7-dihydro-2H-isoindole

### Stade A : Cis-2 -cyano-4,5-cyclohexène-carboxylate de tert-Butyle

On procède comme dans le stade A de l'exemple 1 en utilisant le cis-4,5-cyclohexène-1,2-dicarboxylique anhydride comme substrat.

### Stade B : 1,3-Diphényl-4,7-dihydro-2H-isoindole

Le produit attendu est obtenu en faisant réagir le composé du stade A selon les conditions du stade B de l'exemple 1 avec le réactif utilisé dans l'exemple 2.

| *Microanalyse élémentaire :* | | | |
|---|---|---|---|
| | *C* | *H* | *N* |
| *% calculé* | *88,44* | *6,18* | *5,15* |
| *% trouvé* | *88,52* | *6,31* | *5,16* |
| **Point de fusion : 138 °C.** | | | |

### Exemple 6 : 1,3-Diphényl-5,6-diméthyl-4,7-dihydro-2H-isoindole

Une solution contenant 4,46 g de 1,2-diméthyl-4,5-dibenzoyl-cyclohexène (préparation 1) et 8,81 g de formiate d'ammonium, dans 70 ml d'éthanol anhydre, est portée au reflux pendant 24 heures. On obtient un précipité qui est filtré, puis dissous dans du dichlorométhane. La phase organique est lavée à l'eau, séchée sur sulfate de calcium et concentrée, permettant d'isoler 4 g du produit attendu.

| Microanalyse élémentaire : | | | |
|---|---|---|---|
| | C | H | N |
| % calculé | 88,25 | 7,07 | 4,60 |
| % trouvé | 87,81 | 7,04 | 4,45 |
| **Point de fusion : 212 °C.** | | | |

### Exemple 7 : 1,3-Diphényl-2,5,6-triméthyl-4,7-dihydro-2H-isoindole

On procède comme dans l'exemple 6 en utilisant comme réactif 10 équivalents d'une solution aqueuse à 40 % de méthylamine en présence de 5 équivalents d'acide acétique.

| *Microanalyse élémentaire :* | | | |
|---|---|---|---|
| | *C* | *H* | *N* |
| *% calculé* | *88,14* | *7,40* | *4,47* |
| *% trouvé* | *88,30* | *7,40* | *4,54* |
| **Point de fusion : 200-201 °C.** | | | |

### Exemple 8 : 1,3-Diphényl-2-benzyloxycarbonylamino-4,7-dihydro-2H-isoindole

On procède comme dans l'exemple 6 en utilisant comme réactif 5 équivalents de benzyloxycarbonyl hydrazine et 5 équivalents d'acide acétique.

| *Microanalyse élémentaire :* | | | |
|---|---|---|---|
| | *C* | *H* | *N* |
| *% calculé* | *79,98* | *5,75* | *6,66* |
| *% trouvé* | *79,62* | *5,86* | *6,56* |
| **Point de fusion : 184 °C.** | | | |

### Exemple 9 : 1,3-Diphényl-5,6-diméthyl-4,5,6,7-tétrahydro-2H-isoindole

1 g du produit obtenu dans l'exemple 6, 1,2 g de formiate d'ammonium et 0,2 g de Pd/C à 10 % dans 70 ml d'éthanol sont portés à reflux pendant 3 heures, puis filtrés et concentrés. Après chromatographie sur gel de silice, on isole le produit attendu (éluant : cyclohexane / acétate d'éthyle : 90 / 10).

| *Microanalyse élémentaire :* | | | |
|---|---|---|---|
| | *C* | *H* | *N* |
| *% calculé* | *87,66* | *7,69* | *4,65* |
| *% trouvé* | *87,44* | *7,57* | *4,45* |
| **Point de fusion : 133 °C.** | | | |

### Exemple 10 : 1,3-Diphényl-2-méthyl-4,5,6,7-tétrahydro-2H-isoindole

### Stade A : 1,3-Diphényl-2-méthyl-4,7-dihydro-2H-isoindole

On procède comme dans l'exemple 7 en utilisant comme substrat le 4,5-dibenzoyl-cyclohexène (préparation 2).

### Stade B : 1,3-Diphényl-2-méhtyl-4,5,6,7 -tétrahydro-2H-isoindole

On procède comme dans l'exemple 9 en utilisant comme substrat le produit du stade A.

| *Microanalyse élémentaire :* | | | |
|---|---|---|---|
| | *C* | *H* | *N* |
| *% calculé* | *87,76* | *7,36* | *4,87* |
| *% trouvé* | *87,96* | *7,22* | *4,88* |
| **Point de fusion : 143 °C.** | | | |

### Exemple 11 : 1,3-Diphényl-4,5,6,7-tétrahydro-2H-2-isoindolamine

On procède comme dans l'exemple 9 en utilisant comme substrat le produit de l'exemple 8.

| *Microanalyse élémentaire :* | | | |
|---|---|---|---|
| | *C* | *H* | *N* |
| *% calculé* | *83,30* | *6,99* | *9,71* |
| *% trouvé* | *83,34* | *7,01* | *9,58* |
| **Point de fusion : 161 °C.** | | | |

### Exemple 12 : 1-Phényl-3-(4-méthylthiophényl)-4,5,6,7-tétrahydro-2H-isoindole

### Stade A : 1-Phényl-3 - (4-méthylthiophényl)-4,7-dihydro-2H-isoindole

On procède comme dans l'exemple 6 en utilisant comme produit de départ le 4-benzoyl-5-(4-méthylthio)benzoyl-cyclohexène.

### Stade B : 1-Phényl-3- (4-méthylthiophényl)-4,5,6,7-tétrahydro-2H-isoindole

On procède comme dans l'exemple 9 sur le produit obtenu au stade A.

| *Microanalyse élémentaire :* | | | | |
|---|---|---|---|---|
| | *C* | *H* | *N* | *S* |
| *% calculé* | *78,95* | *6,63* | *4,38* | *10,04* |
| *% trouvé* | *78,91* | *6,61* | *4,39* | *9,96* |
| **Point de fusion : 150 °C.** | | | | |

### Exemple 13 : 1,3-Diphényl-4,7-méthano-4,5,6,7-tétrahydro-2H-isoindole

### Stade A : 1,3-Diphényl-4,7-dihydro-4,7-méthano-2H-isoindole

On procède selon l'exemple 6 en utilisant comme substrat le produit de la préparation 3.

### Stade B : 1,3-Diphényl-4,7-méthane-4,5,6,7-tétrahydro-2H-isoindole

On procède comme dans l'exemple 9 en utilisant le produit du stade A.

| *Microanalyse élémentaire :* | | | |
|---|---|---|---|
| | *C* | *H* | *N* |
| *% calculé* | *88,38* | *6,71* | *4,91* |
| *% trouvé* | *88,44* | *6,96* | *4,73* |
| **Point de fusion : 162°C.** | | | |

### Exemple 14 : 1,3-Diphényl-2-diméthanesulfonylamino-4,5,6,7-tétrahydro-2H-isoindole

La 1,3-Diphényl-4,5,6,7-tétrahydro-2*H*-2-isoindolamine (exemple 11) est traité par 2 équivalents de chlorure de méthanesulfonyle.

| *Microanalyse élémentaire :* | | | | |
|---|---|---|---|---|
| | *C* | *H* | *N* | *S* |
| *% calculé* | *59,44* | *5,44* | *6,30* | *14,42* |
| *% trouvé* | *59,83* | *5,77* | *6,31* | *14,13* |
| **Point de fusion : > 250°C (décomposition).** | | | | |

### Exemple 15 : 1,3-di - (4-Fluorophényl) -2,4,5,6-tétrahydrocyclopenta[c]pyrrole

Le produit attendu est obtenu en faisant réagir le cis-2-cyano-cyclopentane carboxylate de tert-butyle (exemple 4, stade A) selon les conditions du stade B de l'exemple 1.

| *Microanalyse élémentaire :* | | | |
|---|---|---|---|
| | *C* | *H* | *N* |
| *% calculé* | *77,27* | *5,12* | *4,74* |
| *% trouvé* | *77,55* | *4,87* | *4,71* |
| **Point de fusion : 170°C.** | | | |

### Exemple 16 : 1,3-di(4-Méthylphényl)-4,5,6,7-tétrahydro-2H-isoindole

Le produit attendu est obtenu selon la procédé décrit dans l'exemple 1 en utilisant au stade

B le bromure de 4-méthylphénylmagnésium en tant que réactif.

| *Microanalyse élémentaire :* | | | |
|---|---|---|---|
| | *C* | *H* | *N* |
| *% calculé* | *87,66* | *7,69* | *4,65* |
| *% trouvé* | *87,78* | *8,02* | *4,76* |
| **Point de fusion : 150°C.** | | | |

### Exemple 17 : 1,3-Diphényl-4,7-dihydro-4,7-éthano-2H-isoindole

Le produit attendu est obtenu en faisant réagir le composé de la préparation 4 selon les conditions de l'exemple 6.

| *Microanalyse élémentaire :* | | | |
|---|---|---|---|
| | *C* | *H* | *N* |
| *% calculé* | *88,85* | *6,44* | *4,71* |
| *% trouvé* | *88,36* | *6,50* | *4,72* |
| **Point de fusion : 224°C.** | | | |

### Exemple 18 : 1,3-Diphényl-4,5,6,7-tétrahydro-4,7-éthano-2H-isoindole

Le produit attendu est obtenu en faisant réagir le composé de l'exemple 17 selon les conditions de l'exemple 9.

| *Microanalyse élémentaire :* | | | |
|---|---|---|---|
| | *C* | *H* | *N* |
| *% calculé* | *88,25* | *7,07* | *4,68* |
| *% trouvé* | *88,10* | *7,29* | *4,62* |
| **Point de fusion : 248°C.** | | | |

### Exemple 19 : 1,3-di-(4-Fluorophényl)-4,7-méthano-4,5,6,7-tétrahydro-2H-isoindole

### Stade A : 1,3 -di -(4-Fluorophényl)-4,7-dihydro-4,7 -méthano-2H-isoindole

On procède selon l'exemple 6 en utilisant comme substrat le produit de la préparation 5.

### Stade B : 1,3-di-(4-Fluorophényl)-4,7-méthano-4,5,6,7-tétrahydro-2H-isoindole

Le produit obtenu dans le stade A est traité dans les conditions de l'exemple 9 en remplaçant le formiate d'ammonium par du cyclohexène.

| *Microanalyse élémentaire :* | | | |
|---|---|---|---|
| | *C* | *H* | *N* |
| *% calculé* | *78,49* | *5,33* | *4,36* |
| *% trouvé* | *78,59* | *5,36* | *4,40* |
| **Point de fusion : 146°C.** | | | |

### Exemple 20 : 1,3-di-(4-Méthoxyphényl)-4,5,6,7-tétrahydro-2H-isoindole

### Stade A : 1,3 -di -(4-Méthoxyphényl)-4,7-dihydro-2H-isoindole

On procède comme dans l'exemple 6, en utilisant comme substrat le 4,5-di-(4-Méthoxybenzoyl)-cyclohexène (préparation 6).

### Stade B : 1,3-di-(4-Méthoxyphényl)-4,5,6,7-tétrahydro-2H-isoindole

On procède comme dans l'exemple 9 en utilisant le produit du stade A.

| *Microanalyse élémentaire :* | | | |
|---|---|---|---|
| | *C* | *H* | *N* |
| *% calculé* | *79,25* | *6,95* | *4,20* |
| *% trouvé* | *78,65* | *6,83* | *4,28* |
| **Point de fusion : 146°C.** | | | |

### Exemple 21 : 1,3-di-(4-Chlorophényl)-4,5,6,7-tétrahydro-2H-isoindole

### Stade A : 1,3-di- (4 -Chlorophényl )-4,7-dihydro-2H-isoindole

On procède comme dans l'exemple 6, en utilisant comme substrat le 4,5-di-(4-chlorobenzoyl)-cyclohexène (préparation 7).

### Stade B : 1,3 -di - (4-Chlorophényl)-4,5,6,7-tétrahydro-2H-isoindole

On procède comme dans l'exemple 19, en utilisant le produit du stade A.

| *Microanalyse élémentaire :* | | | | |
|---|---|---|---|---|
| | *C* | *H* | *N* | *CI* |
| *% calculé* | *70,19* | *5,01* | *4,09* | *20,72* |
| *% trouvé* | *70,03* | *5,07* | *4,20* | *20,22* |
| **Point de fusion : 321°C** | | | | |

### Exemple 22 : 1,3-Diphényl-2-carboxyméthyl-4,5,6,7-tétrahydro-2H-isoindole

### Stade A : 1,3-Diphényl-2-tert-butoxycarboxylméthyl-4,7-dihydro-2H-isoindole

On procède comme dans l'exemple 6, en utilisant comme substrat le 4,5-Dibenzoyl-cyclohexène (préparation 2)et comme réactif la t-butyl-2-aminoacétate en présence d'acide acétique.

### Stade B : 1,3-Diphényl-2-carboxyméthyl-4,7-dihydro-2H-isoindole

A une solution de 5 g du composé du stade A, dans 50 ml de dichlorométhane anhydre, sont additionnés, à 0°C, 5 ml d'acide trifluoroacétique. Après 12 heures de réaction à température ambiante, le mélange réactionnel est concentré sous vide et le résidu obtenu est rincé à l'éther permettant d'isoler le produit attendu.

### Stade C : 1,3 -Diphényl-2-carboxyméthyl-4,5,6,7-tétrahydro-2H-isoindole

On procède comme dans l'exemple 9, en utilisant le produit du stade B.

| *Microanalyse élémentaire :* | | | |
|---|---|---|---|
| | *C* | *H* | *N* |
| *% calculé* | *79,73* | *6,39* | *4,23* |
| *% trouvé* | *79,72* | *6,58* | *4,19* |
| **Point de fusion : 162°C.** | | | |

### Exemple 23 : (d,l)-1-(4-Fluorophényl)-3-[(4-méthylsulfonyl)phényl]-4,7-méthano-4,5,6,7-tétrahydro-2H-isoindole

On procède comme dans l'exemple 6 en utilisant comme substrat le produit de la préparation 8.

| *Microanalyse élémentaire :* | | | | |
|---|---|---|---|---|
| | *C* | *H* | *N* | *S* |
| *% calculé* | *69,27* | *5,28* | *3,67* | *8,41* |
| *% trouvé* | *69,16* | *5,59* | *3,56* | *7,94* |
| **Point de fusion : > 260°C.** | | | | |

### Exemple 24 : 2-Benzyl-4,6-diphényl-1,2,3,5-tétrahydropyrrolo[3,4-c]pyrrole

On procède comme dans l'exemple 6 en utilisant comme substrat le 3,4-Dibenzoyl-1-benzyl-2,3,4,5-tétrahydro-1*H*-pyrrole;

| *Microanalyse élémentaire :* | | | |
|---|---|---|---|
| | *C* | *H* | *N* |
| *% calculé* | *85,68* | *6,33* | *7,99* |
| *% trouvé* | *85,53* | *6,46* | *7,97* |
| **Point de fusion : 182°C.** | | | |

### Exemple 25 : 1,3-di[(4-Méthylsulfonyl)phényl]-4,7-méthano-4,5,6,7-tétrahydro-2H-isoindole

On procède comme dans l'exemple 6 en utilisant comme substrat le produit de la préparation 9.

| *Microanalyse élémentaire :* | | | | |
|---|---|---|---|---|
| | *C* | *H* | *N* | *S* |
| *% calculé* | *62,56* | *5,25* | *3,17* | *14,52* |
| *% trouvé* | *61,93* | *5,64* | *3,17* | *14,14* |
| **Point de fusion : > 260°C.** | | | | |

### Exemple 26 : Dichlorhydrate de 1,3-di-[4-(1H-imidazolyl)phényl]-4,5,6,7-tétrahydro-2H-isoindole

### Stade A : 1,3-di-[4-(1H-Imidazolyl)phényl]-4,7-dihydro-2H-isoindole

On procède comme dans l'exemple 6 en utilisant comme substrat le produit de la préparation 10.

### Stade B : Dichlorhydrate de 1,3-di-[4-(1H-imidazolyl)phényl]-4,5,6,7-tétrahydro-2H-isoindole

On procède comme dans l'exemple 9 en utilisant le produit du stade A.

| *Microanalyse élémentaire :* | | | | |
|---|---|---|---|---|
| | *C* | *H* | *N* | *Cl* |
| *% calculé* | *65,27* | *5,27* | *14,64* | *14,82* |
| *% trouvé* | *65,38* | *5,59* | *14,71* | *15,30* |
| **Point de fusion : > 260°C.** | | | | |

### Exemple 27 : Chlorhydrate de 1,3-Diphényl-2H-pyrrolidine[3,4-c]pyrrole

On procède comme dans l'exemple 9 en utilisant comme substrat le produit obtenu dans l'exemple 24.
**Point de fusion : > 260°C.**

### Exemple 28 : 1-(4-Fluorophényl)-3-[4-(1H-imidazolyl)phényl]-4,5,6,7-tétrahydro-2H-isoindole

On procède comme dans l'exemple 6 en utilisant comme substrat le 2-(4-Fluorobenzoyl)-1-[4-(1*H*-imidazolyl)benzoyl]cyclohexane.

| *Microanalyse élémentaire :* | | | |
|---|---|---|---|
| | *C* | *H* | *N* |
| *% calculé* | *77,29* | *5,64* | *11,76* |
| *% trouvé* | *76,76* | *5,72* | *11,72* |
| **Point de fusion : > 250°C.** | | | |

### Exemple 29 : 1,3-di-(4-Fluorophényl)-4,7-dihydro-4,7-méthano-2H-isoindole

On procède comme dans l'exemple 19 en s'arrêtant au stade A.

| *Microanalyse élémentaire :* | | | |
|---|---|---|---|
| | *C* | *H* | *N* |
| *% calculé* | *78,98* | *4,73* | *4,39* |
| *% trouvé* | *79,30* | *4,76* | *4,42* |
| **Point de fusion : 189°C.** | | | |

### Exemple 30 : 1,3-Diphényl-2-méthanesulfonylamino-4,5,6,7-tétrahydro-2H-isoindole

On procède comme dans l'exemple 14 en utilisant le même substrat, mais un seul équivalent de chlorure de méthanesulfonyle en tant que réactif.

| *Microanalyse élémentaire :* | | | | |
|---|---|---|---|---|
| | *C* | *H* | *N* | *S* |
| *% calculé* | *68,83* | *6,05* | *7,64* | *8,75* |
| *% trouvé* | *68,94* | *6,32* | *7,63* | *9,07* |
| **Point de fusion : 196°C.** | | | | |

### Exemple 31 : Dichlorhydrate du 1,3-di-(4-Pyridyl)-4,7-méthano-4,5,6,7-tétrahydro-2H-isoindole

On procède comme dans l'exemple 19, des stades A à B, en utilisant comme substrat au stade A le produit de la préparation 11.

| *Microanalyse élémentaire :* | | | | |
|---|---|---|---|---|
| | *C* | *H* | *N* | *Cl* |
| *% calculé* | *63,34* | *5,32* | *11,66* | *19,68* |
| *% trouvé* | *63,69* | *5,67* | *11,73* | *19,44* |
| **Point de fusion : 264°C.** | | | | |

### Exemple 32 : 1,3-di-(2,4-Difluorophényl)-4,7-méthano-4,5,6,7-tétrahydro-2H-isoindole

On procède comme dans l'exemple 19, des stades A à B, en utilisant comme substrat au stade A, le produit de la préparation 12.

### Exemple 33 : 1,3-di-(3,4-Difluorophényl)-4,7-méthano-4,5,6,7-tétrahydro-2H-isoindole

On procède comme dans l'exemple 19, des stades A à B, en utilisant comme substrat, au stade A, le produit de la préparation 13.

### Exemple 34 : 1,3-di-(5-Fluoro-2-pyridyl)-4,7-méthano-4,5,6,7-tétrahydro-2H-isoindole

On procède comme dans l'exemple 19, des stades A à B, en utilisant comme substrat au stade A, le produit de la préparation 14.

### Exemple 35 : 1,3-di-(6-Fluoro-3-pyridyl)-4,7-méthano-4,5,6,7-tétrahydro-2H-isoindole

On procède comme dans l'exemple 19, des stades A à B, en utilisant comme substrat au stade A, le produit de la préparation 15.

### Exemple 36 : 1,3-di-(2-Furyl)-4,7-méthano-4,5,6,7-tétrahydro-2H-isoindole

On procède comme dans l'exemple 19, des stades A à B, en utilisant comme substrat au stade A, le produit de la préparation 16.

### Exemple 37 : 1,3-di-(2-Thiényl)-4,7-méthano-4,5,6,7-tétrahydro-2H-isoindole

On procède comme dans l'exemple 19, des stades A à B, en utilisant comme substrat au stade A, le produit de la préparation 17.

### Exemple 38 : 1,3-di-(2-Amino-4-fluorophényl)-4,7-méthano-4,5,6,7-tétrahydro-2H-isoindole

### Stade A : 1,3-di-(4-Fluoro-2-nitrophényl)-4,7-méthano-4,5,67-tétrahydro-2H-isoindole

On procède comme dans l'exemple 19 des stades A à B, en utilisant au stade A, comme substrat, le produit de la préparation 18.

### Stade B : 1,3-di-(2-Amino-4-fluorophényl)-4,7-méthano-4,5,6,7-tétrahydro-2H-isoindole

Une solution contenant 1 équivalent du composé obtenu dans le stade A, dans 30 ml de méthanol et 100 mg de Palladium sur charbon à 10 %, est chauffée pendant 2 heures à 40°C. Après retour à température ambiante, le milieu réactionnel est filtré sur célite, puis concentré sous pression réduite, permettant d'isoler le produit attendu.

### Exemple 39 : 1,3-di-(4-Fluoro-2-(N-méthyl)aminophényl)-4,7-méthano-4,5,6,7-tétrahydro-2H-isoindole

Une solution contenant 1 équivalent du composé obtenu dans l'exemple 38, est mis à réagir à température ambiante dans du formaldéhyde en présence d'acide formique, selon les conditions décrites dans *Org. React*., 1949, 5, 290, permettant d'isoler le produit attendu.

### Exemple 40 : 1,3-di-(4-Fluoro-2-diméthylaminophényl)-4,7-méthano-4,5,6,7-tétrahydro-2H-isoindole

Une solution contenant 1 équivalent du composé obtenu dans l'exemple 38, 2,2 équivalents d'iodure de méthyle et 2 équivalents de K₂CO₃ dans 40 ml de diméthylformamide est agitée 12 heures à température ambiante. Après concentration sous pression réduite, le résidu est dilué dans du dichlorométhane, puis la phase organique est lavée par une solution saturée en NaCI. Après séchage sur sulfate de sodium et concentration sous pression réduite, une chromatographie sur gel de silice permet d'isoler le produit attendu.

### Exemple 41 : 1,3-di-(3-Amino-4-fluorophényl)-4,7-méthano-4,5,6,7-tétrahydro-2H-isoindole

On procède comme dans l'exemple 38 des stades A et B en utilisant comme substrat au stade A le produit obtenu dans la préparation 19.

### Exemple 42 : 1,3-di-(4-Fluoro-3-(N-méthyl)aminophényl)-4,7-méthano-4,5,6,7-2H-isoindole

On procède comme dans l'exemple 39 en utilisant comme substrat le produit obtenu dans l'exemple 41.
**Point de fusion : 196 °C.**

### Exemple 43 : 1,3-di-(4-Fluoro-3-diméthylaminophényl)-4,7-méthano-4,5,6,7-tétrahydro-2H-isoindole

On procède comme dans l'exemple 40 en utilisant comme substrat le produit obtenu dans l'exemple 41.

### Exemple 44 : 1,3-di-(4-Fluorophényl)-4,5,6,7-tétrahydro-4,7-éthano-2H-isoindole

Le produit attendu est obtenu en faisant réagir le composé de la préparation 20 selon les conditions de l'exemple 6, puis celles de l'exemple 9.

### Exemple 45 : 1,3-di-(3-Acétamide-4-Fluorophényl)-4,7-méthano-4,5,6,7-tétrahdyro-2H-isoindole

Le produit attendu est obtenu en faisant réagir le composé de l'exemple 41 dans des conditions classiques d'acylation.
**Point de fusion : 225°C.**

### Exemple 46 : 5,7-di-(4-Fluorophényl)-1,2,3,4-tétrahydro-1,4-époxy-6H-isoindole

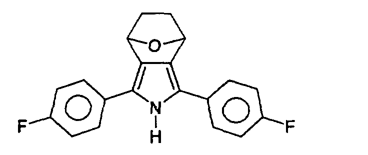

On procède comme dans l'exemple 19, des stades A à B, en utilisant comme substrat au stade A, le produit de la préparation 21.

### ETUDE PHARMACOLOGIQUE DES COMPOSES DE L'INVENTION

### Exemple 47 : Activité biologique des composés sur les cyclo-oxygénases 1 et 2

Les activités inhibitrices des composés pour la COX 1 et la COX 2 ont été évaluées sur macrophages péritonéaux de souris exposés à des stimuli inflammatoires (respectivement zymosan et lipopolysaccharide (LPS)) selon la méthode décrite par Tordjman et al., *Biochimica et Biophysica Acta*, 1995, 1256, 249-56.

Les résultats apparaissent dans la Table 1 :

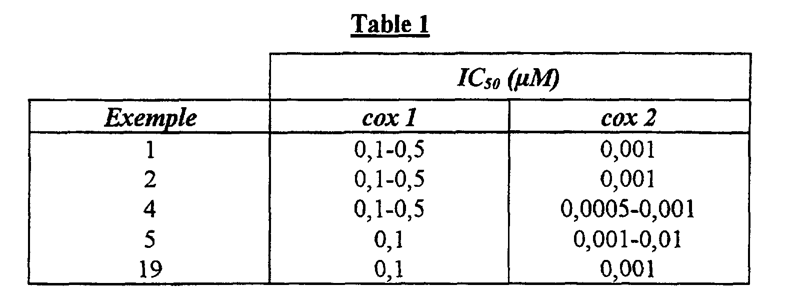

Les composés inhibent également la production d'IL1β des cellules de la lignée THP1 stimulées au LPS. Par exemple, le composé de l'exemple 9 a sur ce modèle une IC₅₀ de l'ordre de 1 µM.
Par ailleurs, ces composés exercent une activité chez l'animal en particulier en inhibant la production de prostaglandines dans le modèle de l'air-pouch à la carragénine chez la souris (Whittle, B.J.R. et al., *Nature*, 1980, 284, 271-273 ; Masferrer, J.L. et al., *Proc. Natl. Acad. Sci. USA*, 1994, 91, 3228-3232). C'est ainsi que le composé de l'exemple 19 présente sur ce modèle une puissante activité avec une ED50 orale de 2,5 mg/kg. La tolérance gastrique, évaluée chez la souris après un jeûne de 24 heures et 5 heures après traitement oral, s'est avérée excellente pour l'ensemble des composés : absence d'atteinte macroscopique jusqu'à la dose de 800 mg/kg.
De plus, les composés de la présente invention inhibe la production de NO chez les macrophages péritonéaux de la souris stimulés au LPS. Par exemple, le composé de l'exemple 19 présente une inhibition dépendante de la concentration avec une IC₅₀ de 2,5 µM.

### EXEMPLE 48 : Formule de préparation pour 1000 comprimés dosés à 10 mg

| | |
|---|---|
| 1,3-di(4-fluorophényle)-4,7-méthano-4,5,6,7-tétrahydro-2H-isoindole | 10 g |
| Amidon de blé | 10 g |
| Lactose | 100 g |
| Stéarate de magnésium | 3 g |
| Silice | 2 g |
| Hydroxypropylcellulose | 2 g |

## Revendications

1. Composés de formule (I) : 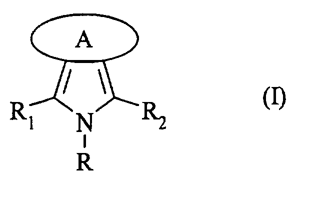 dans laquelle :
**R** représente :
. un atome d'hydrogène,
. un groupement alkyle (C₁-C₆) linéaire ou ramifié, éventuellement substitué par un ou plusieurs groupements, identiques ou différents, choisis parmi hydroxy, alkoxy (C₁-C₆) linéaire ou ramifié, carboxy et alkoxycarbonyle (C₁-C₆) linéaire ou ramifié,
. un groupement amino éventuellement substitué par un ou deux groupements, identiques ou différents, alkyle (C₁-C₆) linéaire ou ramifié, alkoxycarbonyle (C₁-C₆) linéaire ou ramifié, arylalkoxy (C₁-C₆) carbonyle linéaire ou ramifié, alkylsulfonyle (C₁-C₆) linéaire ou ramifié, ou arylsulfonyle,
. ou un groupement acyle (C₁-C₆) linéaire ou ramifié,
**R**_{**1**}**, R**_{**2**} identiques ou différents, représentent chacun indépendamment l'un de l'autre un groupement aryle, ou hétéroaryle, chacun de ces groupements pouvant être éventuellement substitué par un ou plusieurs groupement, identiques ou différents, choisis parmi :
- atome d'halogène,
- groupement alkyle (C₁-C₆) linéaire ou ramifié, éventuellement substitué par un ou plusieurs atomes d'halogène, groupement hydroxy, alkoxy (C₁-C₆) linéaire ou ramifié, amino, ou alkoxycarbonyl (C₁-C₆) linéaire ou ramifié,
- groupement alkoxy (C₁-C₆) linéaire ou ramifié éventuellement substitué par un groupement amino, lui-même éventuellement substitué par un ou deux groupements, identiques ou différents, alkyle (C₁-C₆) linéaire ou ramifié,
- groupement trihalogénoalkoxy (C₁-C₆) linéaire ou ramifié,
- groupement acyle (C₁-C₆) linéaire ou ramifié,
- groupement hydroxy, nitro, cyano, mercapto, carboxy,
- groupement amino éventuellement substitué par un ou deux groupements, identiques ou différents, choisis parmi alkyle (C₁-C₆) linéaire ou ramifié, alkylcarbonyle (C₁-C₆) linéaire ou ramifié, et alkylsulfonyle (C₁-C₆) linéaire ou ramifié,
- groupement alkoxycarbonyle (C₁-C₆) linéaire ou ramifié,
- groupement alkylthio (C₁-C₆) linéaire ou ramifié,
- groupement sulfonyle substitué par un groupement alkyle (C₁-C₆) linéaire ou ramifié ou un groupement amino lui-même éventuellement substitué par un ou deux groupements alkyle (C₁-C₆) linéaire ou ramifié, identiques ou différents,
- et un hétérocycle de 5 à 10 chaînons, monocyclique ou bicyclique, saturé ou insaturé, et contenant 1, 2 ou 3 hétéroatomes, identiques ou différents, choisis parmi azote, oxygène et soufre, éventuellement substitué par un ou plusieurs groupements, identiques ou différents, choisis parmi halogène, alkyle (C₁-C₆) linéaire ou ramifié, et alkoxy (C₁-C₆) linéaire ou ramifié,
**A** représente avec les atomes communs du pyrrole :
- un cycloalkyle (C₃-C₁₂), monocyclique ou bicyclique, saturé ou insaturé (à la condition que la(les) insaturation(s) présente(s) dans le cycloalkyle, ne lui confère(nt) pas un caractère aromatique),
- un hétérocycle saturé de 5 à 7 chaînons contenant un ou deux atomes d'azote,
- ou un oxa-7-bicyclo[2.2.1]heptane, chacun de ces cycles pouvant être éventuellement substitué par un ou plusieurs groupements, identiques ou différents, choisis parmi :
- atome d'halogène,
- groupement trihalogénométhyle,
- groupement alkyle (C₁-C₆) linéaire ou ramifié, éventuellement substitué par un ou plusieurs atomes d'halogène, ou groupements hydroxy,
- groupement alkoxy (C₁-C₆) linéaire ou ramifié,
- groupement aminoalkyle (C₁-C₆) linéaire ou ramifié,
- groupement cyano,
- groupement aryle, arylalkyle (C₁-C₆) linéaire ou ramifié, la partie aryle de cesdits groupements pouvant être éventuellement substituée par un ou plusieurs groupements, identiques ou différents, choisis parmi halogène, hydroxy, alkyle (C₁-C₆) linéaire ou ramifié, et alkoxy (C₁-C₆) linéaire ou ramifié,
- et groupement sulfonyle substitué par un groupement alkyle (C₁-C₆) linéaire ou ramifié ou un groupement amino lui-même éventuellement substitué par un ou deux groupements alkyles (C₁-C₆) linéaires ou ramifiés, identiques ou différents,
ainsi que leurs sels d'addition à un acide ou à une base pharmaceutiquement acceptable,
étant entendu que :
- par groupement aryle, on entend un groupement phényle ou naphtyle, et par groupement hétérorayle, on entend un groupement aryle contenant un, deux ou trois hétéroatomes, d'azote,
étant entendu que :
- lorsque A, avec les atomes communs du cycle pyrrole, consiste en un cyclohexane ou un norbomène et que R consiste en un atome d'hydrogène, alors R₁ et R₂ ne peuvent pas représenter simultanément un groupement phényle,
- lorsque R₁ représente un groupement phényle, R représente un atome d'hydrogène,
□ et que R₂ représente un groupement paranitrophényle, alors A, avec les atomes communs du cycle pyrrole, ne peut pas représenter un norbornane,
□ ou que R₂ représente un groupement 1-isoquinolyle, alors A, avec les atomes communs du cycle pyrrole, ne peut pas représenter un cyclohexane ou un groupement 1,2-indanyle,
et étant entendu également que :
- si R₂ représente un groupement 4-pyridyle ou 4-quinolyle, chacun de ces groupements étant éventuellement substitué,
- et R₁ représente un groupement phényle, naphtyle, pyridyle ou quinolyle, chacun de ces groupements étant éventuellement substitué,
- alors A, avec les atomes communs du cycle pyrrole, ne peut pas représenter un groupement cycloalkyle (C₅-C₈) monocyclique, saturé, ou un hétérocycle saturé de 5 à 7 chaînons contenant 1 ou 2 atomes d'azote, chacun de ces groupements étant éventuellement substitué.

2. Composés de formule (I) selon la revendication 1 **caractérisé en ce que** :
- les groupements R₁ et R₂, identiques ou différents, représentent chacun indépendamment l'un de l'autre, un groupement aryle éventuellement substitué par un ou plusieurs substituant tels que définis dans la revendication 1,
- et A représente, avec les atomes communs du cycle pyrrole, un cycloalkyle (C₃-C₁₂), monocyclique ou bicyclique, saturé ou insaturé mais sans caractère aromatique, éventuellement substitué par un ou plusieurs substituants tels que définis dans la revendications 1,
leurs isomères ainsi que leurs sels d'addition à un acide ou à une base pharmaceutiquement acceptable.

3. Composés de formule (I) selon l'une quelconque des revendications 1 et 2 **caractérisés en ce que** les groupements R₁ et R₂, identiques ou différents, représentent chacun indépendamment l'un de l'autre, un groupement aryle substitué par un ou plusieurs atomes d'halogène,
leurs isomères, ainsi que leurs sels d'addition à un acide ou à une base pharmaceutiquement acceptable.

4. Composés de formule (I) selon l'un quelconque des revendications 1 et 2 **caractérisés en ce que** A représente, avec les atomes communs du cycle pyrrole, un cycloalkyle (C₅-C₈), monocyclique ou bicyclique, saturé ou insaturé mais sans caractère aromatique, éventuellement substitué par un ou plusieurs substituants tels que définis dans la revendication 1,
leurs isomères, ainsi que leurs sels d'addition à un acide ou à une base pharmaceutiquement acceptable.

5. Composés de formule (I) selon l'une quelconque des revendications 1 à 2 **caractérisés en ce que** A représente, avec les atomes communs du cycle pyrrole, un cycloalkyle (C₅-C₁₂) bicyclique, saturé ou insaturé, mais sans caractère aromatique, éventuellement substitué par un ou plusieurs groupements tels que définis dans la revendication 1,
leurs isomères ainsi que leurs sels d'addition à un acide ou à une base pharmaceutiquement acceptable.

6. Composés de formule (I) selon l'une quelconque des revendications 1, 2, 4 et 5 **caractérisés en ce que** A représente, avec les atomes communs du cycle pyrrole, un cycloalkyle (C₅-C₈) bicyclique, saturé ou insaturé, mais sans caractère aromatique, éventuellement substitué par un ou plusieurs groupements tels que définis dans la revendication 1,
leurs isomères, ainsi que leurs sels d'addition à un acide ou à une base pharmaceutiquement acceptable.

7. Composés de formule (I) selon les revendications 1 et 5, **caractérisés en ce que** :
- R₁ et R₂, identiques, représentent un groupement hétéroaryle éventuellement substitué par un ou plusieurs groupements tels que définis dans la revendication 1,
- et A représente, avec les atomes communs du cycle pyrrole, un cycloalkyle (C₅-C₁₂) bicyclique, saturé ou insaturé, mais sans caractère aromatique, éventuellement substitué par un ou plusieurs groupements tels que définis dans la revendication 1,
leurs isomères et leurs sels d'addition à un acide ou à une base pharmaceutiquement acceptable.

8. Composés de formule (I) selon la revendication 7 **caractérisés en ce que** A représente, avec les atomes communs du cycle pyrrole, un cycloalkyle (C₅-C₈) bicyclique, saturé ou insaturé, mais sans caractère aromatique, éventuellement substitué par un ou plusieurs groupements tels que définis dans la revendication 1,
leurs isomères, ainsi que leurs sels d'addition à un acide ou à une base pharmaceutiquement acceptable.

9. Composé de formule (I) selon la revendication 1 qui est le 1,3-di-(4-Fluorophényl)-4,5,6,7-tétrahydro-2*H*-isoindole.

10. Composé de formule (I) selon la revendication 1 qui est le 1,3-Diphényl-5,6-diméthyl-4,5,6,7-tétrahydro-2*H*-isoindole.

11. Composé de formule (I) selon la revendication 1 qui est le 1,3-Diphényl-4,7-méthano-4,5,6,7-tétrahydro-2*H*-isoindole.

12. Composé de formule (I) selon la revendication 1 qui est le 1,3-di-(4-Fluorophényl)-2,4,5,6-tétrahydrocyclopenta[*c*]pyrrole.

13. Composé de formule (I) selon la revendication 1 qui est le 1,3-Diphényl-4,5,6,7-tétrahydro-4,7-éthano-2*H*-isoindole.

14. Composé de formule (I) selon la revendication 1 qui est le 1,3-di-(4-Fluorophényl)-4,7-méthano-4,5,6,7-tétrahydro-2*H*-isoindole.

15. Procédé de préparation des composés de formule (I) selon la revendication 1, **caractérisé en ce que** l'on utilise comme produit de départ :
* *soit des composés de formule (II*/*a) :* 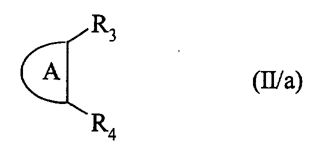 dans laquelle :
A a la même signification que dans la formule (I),
R₃ représente un groupement cyano,
R₄ représente un groupement alkoxycarbonyle (C₁-C₆) linéaire ou ramifié,
composés de formule (II/a) que l'on soumet à l'action d'un magnésien de formule (III) :
R₁ Mg Y (III)
dans laquelle :
R₁ est tel que défini dans la formule (I),
et Y représente un atome d'halogène tel que le brome ou le chlore,
pour conduire aux composés de formule (I/a), cas particulier des composés de formule (I) : 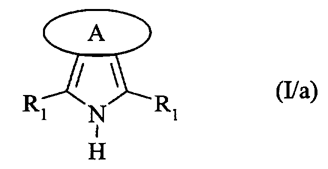 dans laquelle R₁ et A ont la même définition que dans la formule (I),
* *soit des composés de formule (II*/*b) :* 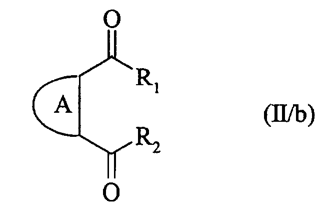 dans laquelle A, R₁ et R₂ sont tels que définis dans la formule (I), que l'on condense :
◆ **soit,** en présence d'un acide organique comme l'acide acétique, avec un composé de formule (IV) :
R-NH₂ (IV)
dans laquelle R a la même signification que dans la formule (I), à la condition que R soit différent d'un atome d'hydrogène,
pour conduire aux composés de formule (I/b), cas particulier des composés de formule (I) : 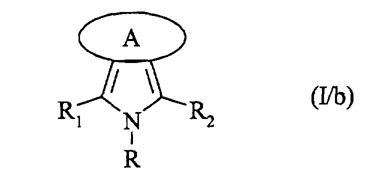 dans laquelle :
R₁ et R₂, identiques ou différents, ont la même signification que dans la formule (I),
A est tel que défini dans la formule (I) et R est tel que défini dans la formule (I) à la condition qu'il ne constitue pas un atome d'hydrogène,
composés de formule (I/b) que l'on soumet éventuellement à l'action d'un agent de déalkylation, de désamination ou de déacylation suivant la nature du groupement R, pour conduire aux composés de formule (I/c), cas particulier des composés de formule (I) : 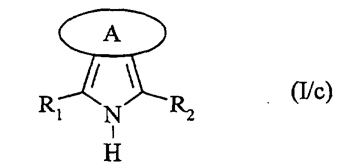 dans laquelle R₁, R₂ et A sont tels que définis précédemment,
◆ **soit** avec du formiate d'ammonium HCO₂⁻ NH₄⁺, pour conduire directement aux composés de formule (I/c), cas particulier des composés de formule (I) : 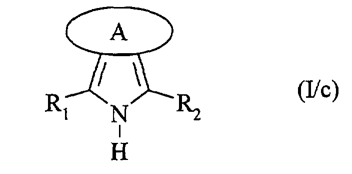 dans laquelle R₁ et R₂ (identiques ou différents), et A ont la même signification que dans la formule (I), l'ensemble des composés (I/a), (I/b) et (I/c) pouvant éventuellement subir une réaction de réduction catalytique, dans les cas où le cycle A comporte au moins une insaturation, pour conduire aux composés de formule (I/d), cas particulier des composés de formule (I) : 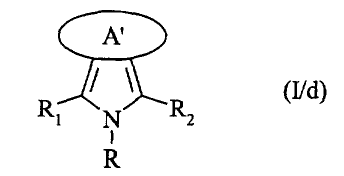 dans laquelle R, R₁ et R₂ ont la même signification que dans la formule (I) et A' représente un cycloalkyle mono ou bicyclique (C₃-C₁₂) saturé (éventuellement substitué),
composés de formule (I/a) à (I/d), que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

16. Compositions pharmaceutiques contenant comme principe actif au moins un composé de formule (I) selon l'une quelconque des revendications 1 à 14, seul ou en combinaison avec un ou plusieurs excipients ou véhicules inertes, non toxiques, pharmaceutiquement acceptables.

17. Compositions pharmaceutiques selon la revendication 16 contenant au moins un principe actif selon l'une quelconque des revendications 1 à 14, utiles comme inhibiteur de la cyclo-oxygénase-2, de l'interleukine 1β et de la nitrique oxyde synthase inductible.

18. Compositions pharmaceutiques contenant comme principe actif au moins un composé de formule (I) selon la revendication 1 dans laquelle :
- R₁ et R₂, identiques, représentent un groupement phényle, R est un atome d'hydrogène, A consiste en un cyclohexane,
- ou R₁ et R₂, identiques, représentent un groupement phényle, R est un atome d'hydrogène, A consiste en un norbornène,
- ou R₁ représente un groupement phényle, R₂ représente un groupement 1-isoquinolyle, R représente un atome d'hydrogène, A consiste en un cyclohexane,
- ou, R₁ représente un groupement phényle, R₂ représente un groupement paranitrophényle, R représente un atome d'hydrogène et A consiste en un norbornane, seul ou en combinaison avec un ou plusieurs excipients ou véhicules inertes, non toxiques, pharmaceutiquement acceptables, pour leurs utilisations comme inhibiteur de la cyclo-oxygénase-2, de l'interleukine 1β et de la nitrique oxyde synthase inductible.

## Patentansprüche

1. Verbindungen der Formel (I): 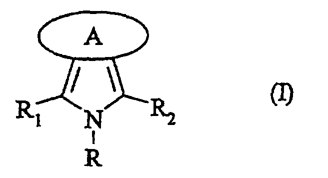 in der:
**R:**
. ein Wasserstoffatom,
. eine geradkettige oder verzweigte (C₁-C₆)-Alkylgruppe, die gegebenenfalls durch eine oder mehrere gleichartige oder verschiedene Gruppen ausgewähl aus Hydroxy, geradkettigem oder verzweigtem (C₁-C₆)-Alkoxy, Carboxy und ge radkettigem oder verzweigtem (C₁-C₆)-Alkoxycarbonyl substituiert ist,
. eine Aminogruppe, die gegebenenfalls durch eine oder zwei gleichartige ode verschiedenartige geradkettige oder verzweigte (C₁-C₆)-Alkylgruppen, gerad kettige oder verzweigte (C₁-C₆)-Alkoxycarbonylgruppen, geradkettige oder ver zweigte Aryl-(C₁-C₆)-alkoxygruppen, geradkettige oder verzweigte (C₁-C₆) Alkylsulfonylgruppen oder Arylsulfonylgruppen substituiert ist, oder eine geradkettige oder verzweigte (C₁-C₆)-Acylgruppe darstellt,
**R**_{**1**} und **R**_{**2**}**,** die gleichartig oder verschieden sind, jeweils unabhängig voneinander eine Aryl- oder Heteroarylgruppe bedeuten, wobei jede dieser Gruppen gegebe nenfalls durch eine oder mehrere gleichartige oder verschiedenartige Grupper substituiert sein kann, ausgewählt aus:
- Halogenatomen,
- geradkettigen oder verzweigten (C₁-C₆)-Alkylgruppen, die gegebenenfalls durch ein oder mehrere Halogenatome, Hydroxygruppen, geradkettige oder verzweigte (C₁-C₆)-Alkoxygruppen, Aminogruppen oder geradkettige oder verzweigte (C₁-C₆)-Alkoxycarbonylgruppen substituiert sind,
- geradkettigen oder verzweigten (C₁-C₆)-Alkoxygruppen, die gegebenenfalls durch eine Aminogruppe substituiert sind, die ihrerseits gegebenenfalls durch eine oder zwei gleichartige oder verschiedene, geradkettige oder verzweigte (C₁-C₆)-Alkylgruppen substituiert ist,
- geradkettigen oder verzweigten (C₁-C₆)-Trihalogenalkoxygruppen,
- geradkettigen oder verzweigten (C₁-C₆)-Acylgruppen,
- Hydroxy-, Nitro-, Cyano-, Mercapto- und Carboxygruppen,
- Aminogruppen, die gegebenenfalls durch eine oder zwei gleichartige oder verschiedenartige Gruppen, ausgewählt aus geradkettigem oder verzweigtem (C₁-C₆)-Alkyl, geradkettigem oder verzweigtem (C₁-C₆)-Alkylcarbonyl und geradkettigem oder verzweigtem (C₁-C₆)-Alkylsulfonylgruppen substituiert sind,
- geradkettigen oder verzweigten (C₁-C₆)-Alkoxycarbonylgruppen,
- geradkettigen oder verzweigten (C₁-C₆)-Alkylthiogruppen,
- Sulfonylgruppen, die durch eine geradkettige oder verzweigte (C₁-C₆)-Alkylgruppe oder eine Aminogruppe substituiert sind, die ihrerseits gegebenenfalls durch eine oder zwei geradkettige oder verzweigte, gleichartige oder verschiedene (C₁-C₆)-Alkylgruppen substituiert ist, und
- monocyclischen oder bicyclischen, gesättigten oder ungesättigten Heterocyclen mit 5 bis 10 Kettengliedern, die 1, 2 oder 3 gleichartige oder verschiedene Heteroatome, ausgewählt aus Stickstoff, Sauerstoff und Schwefel enthalten und gegebenenfalls durch eine oder mehrere gleichartige oder verschiedenartige Gruppen, ausgewählt aus Halogen, geradkettigem oder verzweigtem (C₁-C₆)-Alkyl und geradkettigem oder verzweigtem (C₁-C₆)-Alkoxy substituiert sind, bedeuten,
**A** zusammen mit den gemeinsamen Atomen des Pyrrolrests:
- eine monocyclische oder bicyclische, gesättigte oder ungesättigte (mit der Maßgabe, daß die Unsättigung(en), die in dem Cycloalkylrest vorhanden ist (sind), diesem nicht einen aromatischen Charakter verleiht (verleihen) (C₃-C₁₂)-Cycloalkylgruppe,
- einen gesättigten Heterocyclus mit 5 bis 7 Kettengliedern, der ein oder zwei Stickstoffatome enthält, oder
- eine Oxa-7-bicyclo[2.2.1]heptangruppe darstellt,
wobei jeder dieser Ringe gegebenenfalls durch eine oder mehrere, gleichartige oder verschiedenartige Gruppen substituiert sein kann, ausgewählt aus:
- Halogenatomen,
- Trihalogenmethylgruppen,
- geradkettigen oder verzweigten (C₁-C₆)-Alkylgruppen, die gegebenenfalls durch ein oder mehrere Halogenatome oder Hydroxygruppen substituiert sind,
- geradkettigen oder verzweigten (C₁-C₆)-Alkoxygruppen,
- geradkettigen oder verzweigten (C₁-C₆)-Aminoalkylgruppen,
- Cyanogruppen,
- Arylgruppen, geradkettigen oder verzweigten Aryl-(C₁-C₆)-alkylgruppen, wo- bei der Arylrest dieser Gruppen gegebenenfalls durch eine oder mehrere, gleichartige oder verschiedenartige Gruppen, ausgewählt aus Halogen, Hydroxy, geradkettigem oder verzweigtem (C₁-C₆)-Alkyl und geradkettigem oder verzweigtem (C₁-C₆)-Alkoxy substituiert sein kann, und
- Sulfonylgruppen, die substituiert sind durch eine geradkettige oder verzweigte (C₁-C₆)-Alkylgruppe oder eine Aminogruppe, die ihrerseits gegebenenfalls durch eine oder zwei geradkettige oder verzweigte, gleichartige oder verschiedene (C₁-C₆)-Alkylgruppen substituiert ist,
sowie deren Additionssalze mit einer pharmazeutisch annehmbaren Säure oder Base, mit der Maßgabe, daß:
- man unter einer Arylgruppe eine Phenyl- oder Naphthylgruppe und unter einer Heteroarylgruppe eine Arylgruppe, die ein, zwei oder drei Heteroatome aufweist, versteht, mit der weiteren Maßgabe, daß:
- wenn A zusammen mit den gemeinsamen Atomen des Pyrrolrings einen Cyclohexanrest oder einen Norbonenrest darstellt und R ein Wasserstoffatom bedeutet, dann R₁ und R₂ nicht gleichzeitig eine Phenylgruppe bedeuten können,
- wenn R₁ eine Phenylgruppe darstellt, R ein Wasserstoffatom bedeutet,
□ und wenn R₂ eine p-Nitrophenylgruppe darstellt, dann A zusammen mit den gemeinsamen Atomen des Pyrrolrings kein Norbornan bedeuten kann,
□ oder wenn R₂ eine 1-Isochinolylgruppe darstellt, A zusammen mit den gemeinsamen Atomen des Pyrrolrings nicht einen Cyclohexanrest oder eine 1,2-Indanylgruppe bedeutet,
und mit der weiteren Maßgabe, daß:
- wenn R₂ eine 4-Pyridyl- oder 4-Chinolyl-gruppe darstellt, jede dieser Gruppen gegebenenfalls substituiert ist
- und R₁ eine Phenyl-, Naphthyl-, Pyridyl- oder Chinolylgruppe bedeutet, wobei jede dieser Gruppen gegebenenfalls substituiert ist,
- dann A zusammen mit den gemeinsamen Atomen des Pyrrolrings nicht eine monocyclische, gesättigte (C₅-C₈)-Cycloalkylgruppe darstellt oder einen gesättigten Heterocyclus mit 5 bis 7 Kettengliedern, der 1 oder 2 Stickstoffatome enthält, wobei jede dieser Gruppen gegebenenfalls substituiert ist.

2. Verbindungen der Formel (I) nach Anspruch 1, **dadurch gekennzeichnet, daß**:
- die gleichartigen oder verschiedenen Gruppen R₁ und R₂ jeweils unabhängig voneinander eine gegebenenfalls durch einen oder mehrere Substituenten, wie sie in Anspruch 1 definiert sind, substituierte Arylgruppe bedeuten, und
- A zusammen mit den gemeinsamen Atomen des Pyrrolrings eine monocyclische oder bicyclische, gesättigte oder ungesättigte (C₃-C₁₂)-Cycloalkylgruppe jedoch ohne aromatischen Charakter, die gegebenenfalls durch einen oder mehrere Substituenten, wie sie in Anspruch 1 definiert worden sind, substituiert ist, bedeutet,
deren Isomere sowie deren Additionssalze mit einer pharmazeutisch annehmbaren Säure oder Base.

3. Verbindungen der Formel (I) nach einem der Ansprüche 1 und 2, **dadurch gekennzeichnet, daß** die gleichartigen oder verschiedenen Gruppen R₁ und R₂ jeweils unabhängig voneinander eine durch ein oder mehrere Halogenatome substituierte Arylgruppe bedeuten,
deren Isomere sowie deren Additionssalze mit einer pharmazeutisch annehmbaren Säure oder Base.

4. Verbindungen der Formel (I) nach einem der Ansprüche 1 und 2, **dadurch gekennzeichnet, daß** A zusammen mit den gemeinsamen Atomen des Pyrrolrings eine monocyclische oder bicyclische, gesättigte oder ungesättigte (C₅-C₈)-Cycloalkylgruppe, jedoch ohne aromatischen Charakter, die gegebenenfalls durch einen oder mehrere Substituenten, wie sie in Anspruch 1 definiert worden sind, substituiert ist, bedeutet,
deren Isomere swie deren Additionssalze mit einer pharmazeutisch annehmbaren Säure oder Base.

5. Verbindungen der Formel (I) nach einem der Ansprüche 1 bis 2, **dadurch gekennzeichnet, daß** A zusammen mit den gemeinsamen Atomen des Pyrrolrings eine bicyclische, gesättigte oder ungesättigte (C₅-C₁₂)-Cycloalkylgruppe, jedoch ohne aromatischen Charakter, die gegebenenfalls durch eine oder mehrere Gruppen, wie sie in Anspruch 1 definiert sind, substituiert ist, bedeutet,
deren Isomere sowie deren Additionssalze mit einer pharmazeutisch annehmbaren Säure oder Base.

6. Verbindungen der Formel (I) nach einem der Ansprüche 1, 2, 4 und 5, **dadurch gekennzeichnet, daß** A zusammen mit den gemeinsamen Atomen des Pyrrolrings eine bicyclische, gesättigte oder ungesättigte (C₅-C₈)-Cycloalkylgruppe, jedoch ohne aromatischen Charakter, die gegebenenfalls durch eine oder mehrere Gruppen, wie sie in Anspruch 1 definiert worden sind, substituiert ist, bedeutet,
deren Isomere sowie deren Additionssalze mit einer pharmazeutisch annehmbaren Säure oder Base.

7. Verbindungen der Formel (I) nach den Ansprüchen 1 und 5, **dadurch gekennzeichnet, daß**:
- R₁ und R₂, die gleichartig sind, eine gegebenenfalls durch eine oder mehrere gruppen, wie sie in Anspruch 1 definiert worden sind, substituierte Heteroarylgruppe bedeuten und
- A zusammen mit den gemeinsamen Atomen des Pyrrolrings eine bicyclische, gesättigte oder ungesättigte (C₅-C₁₂)-Cycloalkylgruppe, jedoch ohne aromatischen Charakter, die gegebenenfalls durch eine oder mehrere Gruppen, wie sie in Anspruch 1 definiert worden sind, substituiert ist, bedeutet.
deren Isomere sowie deren Additionssalze mit einer pharmazeutisch annehmbaren Säure oder Base.

8. Verbindungen der Formel (I) nach Anspruch 7, **dadurch gekennzeichnet, daß** A zusammen mit den gemeinsamen Atomen des Pyrrolrings eine bicyclische, gesättigte oder ungesättigte (C₅-C₈)-Cycloalkylgruppe, jedoch ohne aromatischen Charakter, die gegebenenfalls durch eine oder mehrere Gruppen, wie sie in Anspruch 1 definiert worden sind, substituiert ist, bedeutet,
deren Isomere sowie deren Additionssalze mit einer pharmazeutisch annehmbaren Säure oder Base.

9. Verbindung der Formel (I) nach Anspruch 1, nämlich 1,3-Di-(4-Fluorphenyl)-4,5,6,7-tetrahydro-2*H*-isoindol.

10. Verbindung der Formel (I) nach Anspruch 1, nämlich 1,3-Diphenyl-5,6-dimethyl-4,5,6,7-tetrahydro-2*H*-isoindol.

11. Verbindung der Formel (I) nach Anspruch 1, nämlich 1,3-Diphenyl-4,7-methano-4,5,6,7-tetrahydro-2*H*-isoindol.

12. Verbindung der Formel (I) nach Anspruch 1, nämlich 1,3-Di-(4-Fluorphenyl)-2,4,5,6-tetrahydrocyclopenta[*c*]-pyrrol.

13. Verbindung der Formel (I) nach Anspruch 1, nämlich 1,3-Diphenyl-4,5,6,7-tetrahydro-4,7-ethano-2*H*-isoindol.

14. Verbindung der Formel (I) nach Anspruch 1, nämlich 1,3-Di-(4-Fluorphenyl)-4,7-methano-4,5,6,7-tetrahydro-2*H*-isoindol,

15. Verfahren zur Herstellung der Verbindungen der Formel (I) nach Anspruch 1, **dadurch gekennzeichnet, daß** man als Ausgangsprodukt:
* *entweder die Verbindungen der Formel (II*/*a)* verwendet: 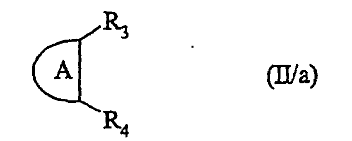 in der:
A die bezüglich der Formel (I) angegebenen Bedeutungen besitzt,
R₃ eine Cyanogruppe darstellt und
R₄ eine geradkettige oder verzweigte (C₁-C₆)-Alkoxycarbonylgruppe bedeutet,
welche Verbindungen der Forme (I) man der Einwirkung einer Magnesiumverbindung der Formel (III) unterwirft:
R₁ Mg Y (III)
in der:
R₁ die bezüglich der Formel (I) angegebenen Bedeutungen besitzt, und
Y ein Halogenatom, wie Brom oder Chlor, bedeutet,
zur Bildung der Verbindungen der Formel (I/a), einem Sonderfall der Verbindungen der Formel (I): 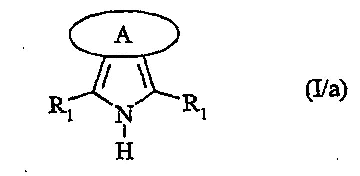 in der R₁ und A die bezüglich der Formel (I) angegebenen Bedeutungen besitzen,
* *oder die Verbindungen der Formel (II*/*b)* verwendet: 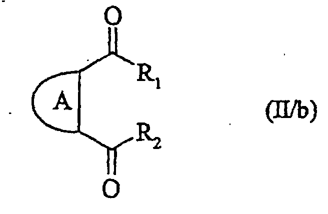 in der A, R₁ und R₂ die bezüglich der Formel (I) angegebenen Bedeutungen besitzen, welche man
◆ **entweder** in Gegenwart einer organischen Säure, wie Essigsäure, mit einer Verbindung der Formel (IV) umsetzt:
R - NH₂ (IV)
in der R die bezüglich der Formel (I) angegebenen Bedeutungen mit der Maßgabe besitzt, daß R von einem Wasserstoffatom verschieden ist, zur Bildung der Verbindungen der Formel (I/b), einem Sonderfall der Verbindungen der Formel (I): 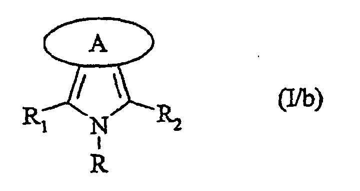 in der:
R₁ und R₂, die gleichartig oder verschieden sind, die bezüglich der Formel (I) angegebenen Bedeutungen besitzen,
A die bezüglich der Formel (I) angegebenen Bedeutungen besitzt und R die bezüglich der Formel (I) angegebenen Bedeutungen mit der Maßgabe, daß es kein Wasserstoffatom darstellt, besitzt,
welche Verbindungen der Formel (I/b) man gegebenenfalls der Einwirkung eines Dealkylierungsmittels, eines Desaminierungsmittels oder eines Deacylierungsmittels in Abhängigkeit von der Art der Gruppe R unterwirft zur Bildung der Verbindungen der Formel (I/c), einem Sonderfall der Verbindungen der Formel (I): 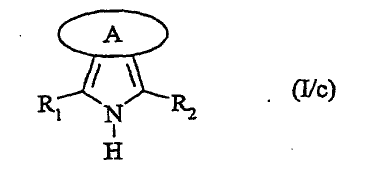 in der R₁, R₂ und A die oben angegebenen Bedeutungen besitzen,
◆ **oder** mit Ammoniumformiat HCO₂⁻ NH₄⁺ umsetzt, so daß man direkt die Verbindungen der Formel (I/c) erhält, einem Sonderfall der Verbindungen der Formel (I): 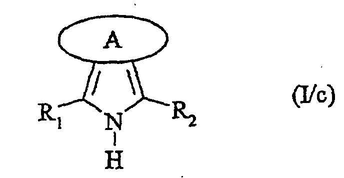 in der R₁ und R₂ (die gleichartig oder verschieden sind) und A die bezüglich der Formel (I) angegebenen Bedeutungen besitzen,
wobei die Gesamtheit der Verbindungen (I/a), (I/b) und (I/c) gegebenenfalls einer katalytischen Reduktion unterworfen werden dann, wenn der Ring A mindestens eine Unsättigung aufweist, zur Bildung der Verbindungen der Formel (I/d), einem Sonderfall der Verbindungen der Formel (I): 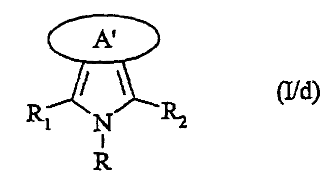 in der R, R₁ und R₂ die bezüglich der Formel (I) angegebenen Bedeutungen besitzen und A' eine mono- oder bicyclische, gesättigte (gegebenenfalls substituierte) (C₃-C₁₂)-Cycloalkylgruppe bedeutet,
welche Verbindungen der Formeln (I/a) bis (I/d) man gegebenenfalls mit Hilfe einer klassischen Reinigungsmethode reinigt, man gegebenenfalls mit Hilfe einer klassischen Trennmethode in die Isomeren auftrennt oder man gewünschtenfalls mit einer pharmazeutisch annehmbaren Säure oder Base in ihre Säureadditionssalze umwandelt.

16. Pharmazeutische Zubereitungen enthalten als Wirkstoff mindestens eine Verbindung der Formel (I) nach einem der Ansprüche 1 bis 14 allein oder in Kombination mit einem oder mehreren inerten, nichttoxischen, pharmazeutisch annehmbaren Träger materialien oder Bindemitteln.

17. Pharmazeutische Zubereitungen nach Anspruch 16 enthaltend mindestens einen Wirkstoff nach einem der Ansprüche 1 bis 14, nützlich als Inhibitor der Cyclo-oxygenase-2, von Interleukin 1β und der inductiblen Stickstoffoxid-synthase.

18. Pharmazeutische Zubereitungen enthaltend als Wirkstoff mindestens eine Verbindung der Formel (I) nach Anspruch 1, worin:
- R₁ und R₂, die gleichartig oder verschieden sind, eine Phenylgruppe, R ein Wasserstoffatom und A einen Cyclohexanrest bedeuten,
- oder R₁ und R₂, die gleichartig oder verschieden sind, eine Phenylgruppe, R ein Wasserstoffatom und A einen Norbornenrest bedeuten,
- oder R₁ eine Phenylgruppe, R₂ eine 1-Isochinolylgruppe, R ein Wasserstoffatom und A einen Cyclohexanrest bedeuten,
- oder R₁ eine Phenylgruppe, R₂ eine p-Nitrophenylgruppe, R ein Wasserstoffatom und A einen Norbornanrest bedeuten, allein oder in Kombination mit einem oder mehreren inerten, nichttoxischen, pharmazeutisch annehmbaren Trägermaterialien oder Bindemitteln zur Verwendung als Inhibitor der Cyclooxygenase-2, von Interleukin 1β und der induktiblen Stickstoffoxid-Synthase.

## Claims

1. Compounds of formula (I) 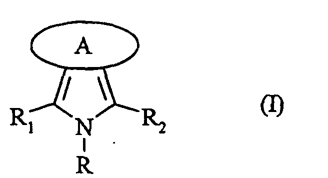 wherein :
**R** represents :
. a hydrogen atom,
. a linear or branched (C₁-C₆)-alkyl group optionally substituted by one or more identical or different groups selected from hydroxy, linear or branched (C₁-C₆)-alkoxy, carboxy and linear or branched (C₁-C₆)-alkoxycarbonyl groups,
. an amino group optionally substituted by one or two identical or different linear or branched (C₁-C₆)-alkyl, linear or branched (C₁-C₆)-alkoxycarbonyl, linear or branched aryl-(C₁-C₆)-alkoxycarbonyl, linear or branched (C₁-C₆)-alkylsulphonyl or arylsulphonyl groups,
. or a linear or branched (C₁-C₆)-acyl group,
**R**_{**1**} **and R**_{**2**}**,** which may be identical or different, each represents independently of the other an aryl or heteroaryl group, it being possible for each of those groups optionally to be substituted by one or more identical or different groups selected from:
- halogen,
- linear or branched (C₁-C₆)-alkyl optionally substituted by one or more halogen atoms or hydroxy, linear or branched (C₁-C₆)-alkoxy, amino or linear or branched (C₁-C₆)-alkoxycarbonyl groups,
- linear or branched (C₁-C₆)-alkoxy optionally substituted by an amino group that is itself optionally substituted by one or two identical or different linear or branched (C₁-C₆)-alkyl groups,
- linear or branched (C₁-C₆)-trihaloalkoxy,
- linear or branched (C₁-C₆)-acyl,
- hydroxy, nitro, cyano, mercapto, carboxy,
- amino optionally substituted by one or two identical or different groups selected from linear or branched (C₁-C₆)-alkyl, linear or branched (C₁-C₆)-alkylcarbonyl and linear or branched (C₁-C₆)-alkylsulphonyl,
- linear or branched (C₁-C₆)-alkoxycarbonyl,
- linear or branched (C₁-C₆)-alkylthio,
- sulphonyl substituted by a linear or branched (C₁-C₆)-alkyl group or by an amino group itself optionally substituted by one or two identical or different linear or branched (C₁-C₆)-alkyl groups,
and
- a saturated or unsaturated, monocyclic or bicyclic heterocycle having from 5 to 10 ring members and containing 1, 2 or 3 identical or different hetero atoms selected from nitrogen, oxygen and sulphur, optionally substituted by one or more identical or different groups selected from halogen, linear or branched (C₁-C₆)-alkyl and linear or branched (C₁-C₆)-alkoxy,
**A** represents, together with the atoms in common with the pyrrole :
- a saturated or unsaturated, monocyclic or bicyclic (C₃-C₁₂)-cycloalkyl group (with the proviso that the unsaturation(s) present in the cycloalkyl does(do) not confer on it an aromatic character),
- a saturated heterocycle having from 5 to 7 ring members and containing one or two nitrogen atoms,
- or a 7-oxabicyclo[2.2.1]heptane radical,
it being possible for each of those rings optionally to be substituted by one or more identical or different groups selected from :
- halogen,
- trihalomethyl,
- linear or branched (C₁-C₆)-alkyl optionally substituted by one or more halogen atoms or hydroxy groups,
- linear or branched (C₁-C₆)-alkoxy,
- linear or branched (C₁-C₆)-aminoalkyl,
- cyano,
- aryl and linear or branched aryl-(C₁-C₆)-alkyl, it being possible for the aryl moiety of the said groups optionally to be substituted by one or more identical or different groups selected from halogen, hydroxy, linear or branched (C₁-C₆)-alkyl and linear or branched (C₁-C₆)-alkoxy,
- and sulphonyl substituted by a linear or branched (C₁-C₆)-alkyl group or by an amino group itself optionally substituted by one or two identical or different linear or branched (C₁-C₆)-alkyl groups,
and also addition salts thereof with a pharmaceutically acceptable acid or base,
wherein an aryl group is to be understood as meaning a phenyl or naphthyl group and a heteroaryl group is to be understood as meaning an aryl group containing one, two or three nitrogen hetero atoms,
provided that :
- when A, together with the atoms in common with the pyrrole ring, is a cyclohexane or a norbornene and R is a hydrogen atom, R₁ and R₂ cannot simultaneously each represent a phenyl group,
- when R₁ represents a phenyl group, R represents a hydrogen atom
□ and when R₂ represents a *para*-nitrophenyl group, then A, together with the atoms in common with the pyrrole ring, cannot represent a norbornane,
□ or when R₂ represents a 1-isoquinolyl group, then A, together with the atoms in common with the pyrrole ring, cannot represent a cyclohexane or a 1,2-indanyl group,
and provided also that :
- if R₂ represents a 4-pyridyl or 4-quinolyl group, each of those groups optionally being substituted,
- and R₁ represents a phenyl, naphthyl, pyridyl or quinolyl group, each of those groups optionally being substituted,
- then A, together with the atoms in common with the pyrrole ring, cannot represent a saturated monocyclic (C₅-C₈)-cycloalkyl group or a saturated heterocycle having from 5
to 7 ring members and containing 1 or 2 nitrogen atoms, each of such groups optionally being substituted.

2. Compounds of formula (I) according to claim 1, **characterised in that** :
- the groups R₁ and R₂, which may be identical or different, each represents independently of the other an aryl group optionally substituted by one or more substituents as defined in claim 1,
- and A represents, together with the atoms in common with the pyrrole ring, a monocyclic or bicyclic (C₃-C₁₂)-cycloalkyl group that is saturated or unsaturated but not of aromatic character, optionally substituted by one or more substituents as defined in claim 1,
their isomers, and also addition salts thereof with a pharmaceutically acceptable acid or base.

3. Compounds of formula (I) according to either claim 1 or claim 2, **characterised in that** the groups R₁ and R₂, which may be identical or different, each represents independently of the other an aryl group substituted by one or more halogen atoms,
their isomers, and also addition salts thereof with a pharmaceutically acceptable acid or base.

4. Compounds of formula (I) according to either claim 1 or claim 2, **characterised in that** A represents, together with the atoms in common with the pyrrole ring, a monocyclic or bicyclic (C₅-C₈)-cycloalkyl group that is saturated or unsaturated but not of aromatic character, optionally substituted by one or more substituents as defined in claim 1,
their isomers, and also addition salts thereof with a pharmaceutically acceptable acid or base.

5. Compounds of formula (I) according to either claim I or claim 2, **characterised in that** A represents, together with the atoms in common with the pyrrole ring, a bicyclic (C₅-C₁₂)-cycloalkyl group that is saturated or unsaturated but not of aromatic character, optionally substituted by one or more groups as defined in claim 1,
their isomers, and also addition salts thereof with a pharmaceutically acceptable acid or base.

6. Compounds of formula (I) according to any one of claims 1, 2, 4 and 5, **characterised in that** A represents, together with the atoms in common with the pyrrole ring, a bicyclic (C₅-C₈)-cycloalkyl group that is saturated or unsaturated but not of aromatic character, optionally substituted by one or more groups as defined in claim 1,
their isomers, and also addition salts thereof with a pharmaceutically acceptable acid or base.

7. Compounds of formula (I) according to claims 1 and 5, **characterised in that** :
- R₁ and R₂, which are identical, each represents a heteroaryl group optionally substituted by one or more groups as defined in claim 1,
- and A represents, together with the atoms in common with the pyrrole ring, a bicyclic (C₅-C₁₂)-cycloalkyl group that is saturated or unsaturated but not of aromatic character, optionally substituted by one or more groups as defined in claim 1,
their isomers, and addition salts thereof with a pharmaceutically acceptable acid or base.

8. Compounds of formula (I) according to claim 7, **characterised in that** A represents, together with the atoms in common with the pyrrole ring, a bicyclic (C₅-C₈)-cycloalkyl group that is saturated or unsaturated but not of aromatic character, optionally substituted by one or more groups as defined in claim 1,
their isomers, and also addition salts thereof with a pharmaceutically acceptable acid or base.

9. Compound of formula (I) according to claim 1, which is 1,3-di-(4-fluorophenyl)-4,5,6,7-tetrahydro-2*H*-isoindole.

10. Compound of formula (I) according to claim 1, which is 1,3-diphenyl-5,6-dimethyl-4,5,6,7-tetrahydro-2*H*-isoindole.

11. Compound of formula (I) according to claim 1, which is 1,3-diphenyl-4,7-methano-4,5,6,7-tetrahydro-2*H*-isoindole.

12. Compound of formula (I) according to claim 1, which is 1,3-di-(4-fluorophenyl)-2,4,5,6-tetrahydrocyclopenta[*c*]pyrrole.

13. Compound of formula (I) according to claim 1, which is 1,3-diphenyl-4,5,6,7-tetrahydro-4,7-ethano-2*H*-isoindole.

14. Compound of formula (I) according to claim 1, which is 1,3-di-(4-fluorophenyl)-4,7-methano-4,5,6,7-tetrahydro-2*H*-isoindole.

15. Process for the preparation of compounds of formula (I) according to claim 1, **characterised in that** there are used as starting material :
* *either compounds of formula (II*/*a) :* 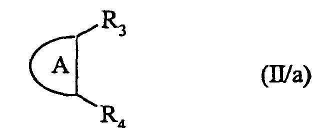 wherein :
A is as defined for formula (I),
R₃ represents a cyano group and
R₄ represents a linear or branched (C₁-C₆)alkoxycarbonyl group,
which compounds of formula (II/a) are subjected to the action of a magnesium compound of formula (III) :
R₁ Mg Y (III)
wherein :
R₁ is as defined for formula (I),
and Y represents a halogen atom, such as bromine or chlorine,
to yield the compounds of formula (I/a), a particular case of the compounds of formula (I) : 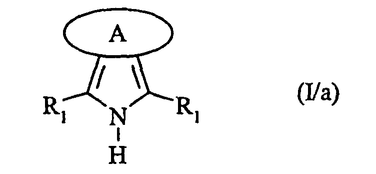 wherein R₁ and A are as defined for formula (I),
* *or compounds of formula (II*/*b) :* 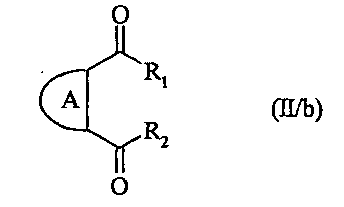 wherein A, R₁ and R₂ are as defined for formula (I),
which are condensed :
◆ **either** in the presence of an organic acid, such as acetic acid, with a compound of formula (IV) :
R-NH₂ (IV)
wherein R is as defined for formula (I), with the proviso that R is other than a hydrogen atom,
to yield the compounds of formula (I/b), a particular case of the compounds of formula (I) : 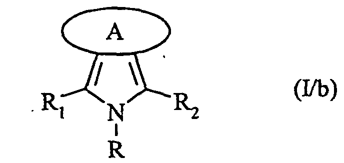 wherein :
R₁ and R₂, which may be identical or different, are as defined for formula (I),
A is as defined for formula (I) and R is as defined for formula (I) with the proviso that it is not a hydrogen atom, which compounds of formula (I/b) are optionally subjected to the action of a dealkylation, deamination or deacylation agent according to the nature of the group R, to yield the compounds of formula (I/c), a particular case of the compounds of formula (I) : 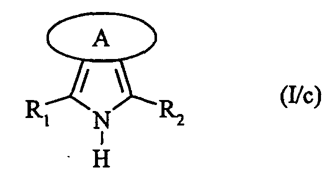
wherein R₁, R₂ and A are as defined hereinabove,
◆ **or** with ammonium formate HCO₂⁻ NH₄⁺ to yield the compounds of formula (I/c) directly, a particular case of the compounds of formula (I): 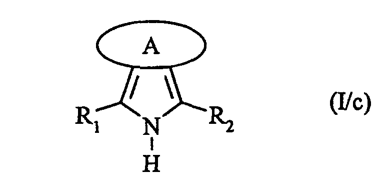 wherein R₁ and R₂ (identical or different) and A are as defined for formula (I),
it being possible for each of the compounds (I/a), (I/b) and (I/c) optionally to be subjected to a catalytic reduction, in the cases where the ring A comprises at least one unsaturation, to yield the compounds of formula (I/d), a particular case of the compounds of formula (I) : 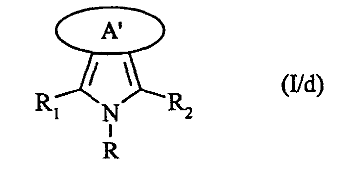 wherein R, R₁ and R₂ are as defined for formula (I) and A' represents an (optionally substituted) saturated, mono- or bi-cyclic (C₃-C₁₂)-cycloalkyl group,
which compounds of formulae (I/a) to (I/d) are purified, if necessary, according to a conventional purification technique, are separated, where appropriate, into their isomers according to a conventional separation technique, and are converted, if desired, into addition salts with a pharmaceutically acceptable acid or base.

16. Pharmaceutical compositions comprising as active ingredient at least one compound of formula (I) according to any one of claims 1 to 14, alone or in combination with one or more pharmaceutically acceptable, inert, non-toxic excipients or carriers.

17. Pharmaceutical compositions according to claim 16 containing at least one active ingredient according to any one of claims 1 to 14, for use as inhibitors of cyclooxygenase-2, interleukin 1β and inducible nitric oxide synthase.

18. Pharmaceutical compositions containing as active ingredient at least one compound of formula (I) according to claim 1 wherein :
- R₁ and R₂, which are identical, each represents a phenyl group, R is a hydrogen atom and A is a cyclohexane,
- or R₁ and R₂, which are identical, each represents a phenyl group, R is a hydrogen atom and A is a norbornene,
- or R₁ represents a phenyl group, R₂ represents a 1-isoquinolyl group, R represents a hydrogen atom and A is a cyclohexane,
- or R₁ represents a phenyl group, R₂ represents a *para*-nitrophenyl group, R represents a hydrogen atom and A is a norbornane,
alone or in combination with one or more pharmaceutically acceptable, inert, non-toxic excipients or carriers, for use as inhibitors of cyclooxygenase-2, interleukin 1β and inducible nitric oxide synthase.
